# EUROPEAN PATENT APPLICATION

(11) **EP 4 707 382 A1**
(43) Date of publication of application: **11.03.2026**
(21) Application number: 24797209.4
(22) Date of filing: 26.04.2024
(51) Int. Cl.: C12N 5/071, C12N 1/00, C12Q 1/02

(54) **METHOD FOR PRODUCING METABOLICALLY ACTIVATED LIVER ORGANOID, COMPOSITION, AND METHOD FOR EVALUATING TEST SUBSTANCE**

(30) Priority: 28.04.2023 JP 2023074944
(71) Applicant: JSR Corporation, Tokyo 105-8640 (JP); Keio University, Tokyo, 108-8345 (JP)
(72) Inventor: OKADA Ryo, Tokyo 105-8640 (JP); SATO Toshiro, Tokyo 160-8582 (JP); IGARASHI Ryo, Tokyo 160-8582 (JP)
(74) Representative: TBK
(86) International application number: PCT/JP2024/016542
(87) International publication number: WO 2024/225466

(57) **Abstract**

A method for producing a metabolically activated liver organoid, the method including a step of culturing primary hepatocytes or hepatocyte-like cells in a differentiation medium to obtain a metabolically activated liver organoid, in which the differentiation medium is a medium including one or two selected from the group consisting of a growth hormone receptor agonist and a prolactin receptor agonist, and a glucocorticoid receptor agonist.

## Description

### [Technical Field]

The present invention relates to a method for producing a metabolically activated liver organoid, a composition, and a method for evaluating a test substance. Priority is claimed on Japanese Patent Application No. 2023-074944, filed on April 28, 2023, the content of which is incorporated herein by reference.

### [Background Art]

Human hepatocytes are an important experimental tool for drug discovery research. Although primary human hepatocytes maintain liver function to a certain extent, there are problems such as ethical issues, difficulty in obtaining the primary human hepatocytes, difficulty in securing quantities required for experimental tools, a decrease in function, and a large lot-to-lot difference, which makes it difficult to use the primary human hepatocytes in drug discovery research. Therefore, attempts have been made to use human liver organoids instead of the primary human hepatocytes as a supply source of human hepatocytes.

Examples of the main functions of hepatocytes include (1) a drug metabolizing function, (2) a transporter function, (3) a bile production function, (4) a protein production function, (5) a lipid metabolizing function, (6) a gluconeogenesis function, and (7) detoxification of ammonia (urea production function). However, up to now, human liver organoids in which the gluconeogenesis function is sufficiently expressed have not been known.

Non-Patent Document 1 describes a human liver organoid obtained by culturing HepG2 (human liver cancer-derived cell line) in a medium including glucagon, and gluconeogenesis thereof.

### [Citation List]

### [Non-Patent Document]

Non-Patent Document 1: Gamboa C. M., et al., Optimized 3D Culture of Hepatic Cells for Liver Organoid Metabolic Assays, Cells, 10 (12), 3280, 2021.

### [Summary of Invention]

### [Technical Problem]

An object of the present invention is to provide technology for producing a metabolically activated liver organoid, in which gluconeogenesis and urea production are activated.

### [Solution to Problem]

The present invention includes the following embodiments.
[1] A method for producing a metabolically activated liver organoid, the method including:
   a step (a) of culturing primary hepatocytes or hepatocyte-like cells in a differentiation medium to obtain a metabolically activated liver organoid,
   in which the differentiation medium is a medium including one or two selected from the group consisting of a growth hormone receptor agonist and a prolactin receptor agonist, and a glucocorticoid receptor agonist.
[2] The method for producing a metabolically activated liver organoid according to [1],
   in which the differentiation medium includes the growth hormone receptor agonist and the prolactin receptor agonist.
[3] The method for producing a metabolically activated liver organoid according to [1] or [2],
   in which the differentiation medium further includes one or two growth factors selected from the group consisting of an epidermal growth factor (EGF), a hepatocyte growth factor (HGF), and a fibroblast growth factor (FGF).
[4] The method for producing a metabolically activated liver organoid according to any one of [1] to [3],
   in which the differentiation medium further includes a transforming growth factor β receptor antagonist.
[5] The method for producing a metabolically activated liver organoid according to any one of [1] to [4],
   in which the differentiation medium further includes a γ-secretase inhibitor.
[6] The method for producing a metabolically activated liver organoid according to any one of [1] to [5],
   in which the differentiation medium further includes one or two or more selected from the group consisting of estradiol, dehydroepiandrosterone, and glucagon.
[7] The method for producing a metabolically activated liver organoid according to any one of [1] to [6], the method further including, in the following order after the step (a):
   a step (b) of fasting the metabolically activated liver organoid; and
   a step (c) of culturing the metabolically activated liver organoid in a gluconeogenesis medium,
   in which the gluconeogenesis medium is a medium including a gluconeogenesis substrate.
[8] The method for producing a metabolically activated liver organoid according to [7],
   in which the gluconeogenesis medium further includes a glucagon receptor agonist.
[9] The method for producing a metabolically activated liver organoid according to [7] or [8],
   in which the gluconeogenesis medium further includes one or two selected from the group consisting of a growth hormone receptor agonist and a prolactin receptor agonist, and a glucocorticoid receptor agonist.
[10] A composition including:
   one or two selected from the group consisting of a growth hormone receptor agonist and a prolactin receptor agonist; and
   a glucocorticoid receptor agonist.
[11] A method for evaluating a test substance, the method including:
   a step of bringing a test substance into contact with a metabolically activated liver organoid produced by the production method according to any one of [1] to [9]; and
   a step of evaluating an effect of the test substance on the metabolically activated liver organoid.

### [Advantageous Effects of Invention]

According to the present invention, it is possible to provide a technique for producing a metabolically activated liver organoid, in which gluconeogenesis and urea production are activated.

### [Brief Description of Drawings]

[FIG. 1] FIG. 1 is a view showing a procedure of Experimental Example 1.
[FIG. 2] FIG. 2 is a view showing a procedure of Experimental Example 2.
[FIG. 3] FIG. 3 is a view showing a procedure of Experimental Example 3.
[FIG. 4] FIG. 4 is a graph showing the amount of albumin produced, as measured in Experimental Examples 1 to 3.
[FIG. 5] FIG. 5 is a graph showing the amount of bile acid produced, as measured in Experimental Examples 1 to 3.
[FIG. 6] FIG. 6 is a graph showing the concentrations of bile acids in general human liver tissue and human serum.
[FIG. 7] FIG. 7 is a graph showing the quantification results of glucose in Experimental Example 5.
[FIG. 8] FIG. 8 is a graph showing the quantification results of glucose in Experimental Example 5.
[FIG. 9] FIG. 9 is a graph showing the quantification results of glucose in Experimental Example 6.
[FIG. 10] FIG. 10 is a graph showing the measurement results of glucose in Experimental Example 7.
[FIG. 11] FIG. 11 is a graph showing the measurement results of urea in Experimental Example 7.
[FIG. 12] FIG. 12 is a view showing a procedure of Experimental Example 8.
[FIG. 13] FIG. 13 is a graph showing the measurement results of CYP metabolites in Experimental Example 8.
[FIG. 14] FIG. 14 is a view showing a procedure of Experimental Example 9.
[FIG. 15] FIG. 15 is a graph showing the measurement results of CYP metabolites in Experimental Example 9.
[FIG. 16] FIG. 16 is a heat map showing the results of Experimental Example 10.
[FIG. 17] FIG. 17 is a view showing a procedure of Experimental Example 11.
[FIG. 18] FIG. 18 is a graph showing the measurement results of urea and glucose in Experimental Example 11.
[FIG. 19] FIG. 19 is a view showing a procedure of Experimental Example 12.
[FIG. 20] FIG. 20 is a graph showing the measurement results of urea and glucose in Experimental Example 12.
[FIG. 21] FIG. 21 is a graph showing the measurement results of urea and glucose in Experimental Example 12.
[FIG. 22] FIG. 22 is a graph showing the measurement results of glycogen in Experimental Example 13.
[FIG. 23] FIG. 23 is a table showing the results of quantitative real-time PCR in Experimental Example 14.
[FIG. 24] FIG. 24 is a graph showing the measurement results of glucose in Experimental Example 15.
[FIG. 25] FIG. 25 is a graph showing the measurement results of glucose in Experimental Example 16.
[FIG. 26] FIG. 26 is a graph showing the results of quantitative real-time PCR in Experimental Example 17.
[FIG. 27] FIG. 27 is a graph showing the measurement results of the amount of secreted protein produced in Experimental Example 18.
[FIG. 28] FIG. 28 is an image showing the results of lipid staining of the liver organoid in Experimental Example 19.
[FIG. 29] FIG. 29 is a graph showing the measurement results of the amount of lipid produced in Experimental Example 19.
[FIG. 30] FIG. 30 is a graph showing the results of the lipoprotein profiling of triglycerides in Experimental Example 19.
[FIG. 31] FIG. 31 is a graph showing the results of the lipoprotein profiling of cholesterol in Experimental Example 19.
[FIG. 32] FIG. 32 is an image showing the results of the periodic acid-Schiff (PAS) staining of the liver organoid in Experimental Example 20.
[FIG. 33] The upper part of FIG. 33 is a graph showing the measurement results of the amount of ATP in Experimental Example 21. The lower part of FIG. 33 is a graph showing the measurement results of the amount of LDH in Experimental Example 21.
[FIG. 34] FIG. 34 is a fluorescence image of a liver organoid captured in Experimental Example 22.

### [Description of Embodiments]

Unless particularly stated otherwise, each component mentioned as an exemplary example in the present specification, for example, each of the components included in the medium and the components used in each step can be used alone or a combination of two or more kinds thereof can be used.

In the present specification, such a notation representing a numerical range as "A to B" has the same meaning as "equal to or more than A and equal to or less than B". In addition, in the present specification, a notation representing a numerical range such as a phrase "A to B, preferably a to b" is the same as the phrases "equal to or more than A and equal to or less than B", "equal to or more than A and equal to or less than b", "equal to or more than a and equal to or less than B", and "equal to or more than a and equal to or less than b".

**In** the present specification, the phrase "medium including a substance X" and "in the presence of a substance X" mean a medium to which an exogenous substance X has been added, a medium including an exogenous substance X, or in the presence of an exogenous substance X. That is, in a case where a cell or tissue present in the medium endogenously expresses, secretes, or produces the substance X, it should be noted that the endogenous substance X is distinguished from the exogenous substance X, and a medium which does not include the exogenous substance X does not fall under the category of "medium including substance X" even when the medium includes the endogenous substance X.

### [Method for Producing Metabolically Activated Liver Organoid]

**In** one embodiment, the present invention provides a method for producing a metabolically activated liver organoid, the method including a step (a) of culturing primary hepatocytes or hepatocyte-like cells in a differentiation medium to obtain a metabolically activated liver organoid, in which the differentiation medium is a medium including one or two selected from the group consisting of a growth hormone receptor agonist and a prolactin receptor agonist, and a glucocorticoid receptor agonist.

In the present specification, the term "organoid" means a self-organized cellular tissue body obtained through cell culture. Furthermore, the liver organoid means an organoid formed from a primary hepatocyte, a pluripotent stem cell, or a hepatic progenitor cell. In addition, the "metabolically activated liver organoid" means a liver organoid that has gluconeogenic ability and urea producing ability equivalent to or more than those of the primary hepatocytes.

According to the production method of the present embodiment, a metabolically activated liver organoid can be produced. As described later in Examples, the metabolically activated liver organoid obtained by the production method of the present embodiment has improved gluconeogenesis and urea production, as compared with the liver organoid in the related art, and has a function similar to that of primary human hepatocytes.

The production method of the present embodiment includes a step of culturing primary hepatocytes or hepatocyte-like cells in a differentiation medium to obtain a metabolically activated liver organoid.

In the production method of the present embodiment, the primary hepatocytes are directly obtained from the liver tissue. As the primary hepatocytes, primary human hepatocytes (PHHs) are preferable. The hepatocyte-like cells refer to cells having a function or form similar to those of the primary hepatocytes. Examples of the hepatocyte-like cells include cells included in a liver organoid. In the production method of the present embodiment, it is preferable to use hepatocyte-like cells, it is more preferable to use a liver organoid, and it is still more preferable to use a human liver organoid.

The primary hepatocytes or hepatocyte-like cells may be those that have been expansion-cultured in an appropriate medium. The medium used for the expansion culture is not particularly limited as long as it is a medium that is usually used for the expansion culture of primary hepatocytes or hepatocyte-like cells. For example, the media described in Gamboa C. M., et al., Optimized 3D Culture of Hepatic Cells for Liver Organoid Metabolic Assays, Cells, 10, 3280, 2021 (which describes a method for culturing an HepG2 cell-derived organoid, in which the medium includes B27 Supplement (without vitamin A), 1: 100 N2 Supplement, 1 mM N-acetylcysteine, 10% (v/v) Rspondin 1 conditioned medium, 10 mM Nicotinamide, 10 nM recombinant human [Leu15]-Gastrin-I, 50 ng/mL recombinant human EGF, 100 ng/mL recombinant human FGF10, and 25 ng/mL recombinant human HGF); Mitani et al., Human ESC/iPSC-Derived Hepatocyte-like Cells Achieve Zone-Specific Hepatic Properties by Modulation of WNT Signaling, Molecular Therapy, 25 (6), 2017 (which describes a method for inducing differentiation into various hepatocyte-like cells having zone-specific characteristics in the liver lobule, in which, for example, an RPMI 1640 medium used in a method for inducing differentiation into hepatocytes includes 1 × B27 Supplement (without vitamin A) (Thermo Fisher Scientific), 1% GlutaMAX (Thermo Fisher Scientific), and 20 ng/mL HGF (R&D Systems)); Peng W. C., et al., Inflammatory Cytokine TNFa Promotes the Long-Term Expansion of Primary Hepatocytes in 3D Culture, Cell 175, 1607-1619, 2018 (which describes a method for expansion-culturing an organoid derived from a hepatocyte, in which the medium includes 3 µM CHIR99021 (Peprotech), 25 ng/mL EGF (Peprotech), 50 ng/mL HGF (Peprotech), 100 ng/mL TNFα (Peprotech), and 50 ng/mL noggin (Peprotech)); Hu H., et al., Long-Term Expansion of Functional Mouse and Human Hepatocytes as 3D Organoids, Cell 175, 1591-1606, 2018 (which describes a method for expansion-culturing an organoid derived from a primary hepatocyte, in which, for example, an expansion-medium for the organoid derived from a primary hepatocyte includes AdDMEM/F12 (Thermo Fisher Scientific, with HEPES, GlutaMax, and Penicillin-Streptomycin) plus 15% RSP01 conditioned medium (homemade), B27 (minus vitamin A), 50 ng/mL EGF (Peprotech), 1.25 mM N-acetylcysteine (Sigma), 10 nM gastrin (Sigma), 3 µM CHIR99021 (Sigma), 50 ng/mL HGF (Peprotech), 100 ng/mL FGF7 (Peprotech), 100 ng/mL FGF10 (Peprotech), 2 µM A83-01 (Tocris), 10 mM Nicotinamide (Sigma), 10 µM rho inhibitor γ-27632 (Calbiochem), and 20 ng/mL TGFα), expansion-media (EM media) used in Examples which will be described later, or the like can be used.

In the production method of the present embodiment, the differentiation medium includes one or two selected from the group consisting of a growth hormone receptor agonist and a prolactin receptor agonist, and a glucocorticoid receptor agonist.

In the present specification, the receptor agonist includes factors that mediate signal transduction to the receptor and a transcription factor downstream of the receptor.

The growth hormone is a hormone secreted from a growth hormone-secreting cell of the anterior lobe of the pituitary gland. It is known that the growth hormone activates STATS. Examples of the growth hormone receptor agonist include a ligand having an agonistic action on the growth hormone receptor. Examples of such a ligand include a growth hormone and a growth hormone mimetic. Examples of the growth hormone mimetic include an agonist antibody, an antibody fragment, and a peptide against the growth hormone receptor. Examples of the NCBI accession number of the cDNA of a human growth hormone include NM_000515.5, NM_022559.4, NM_022560.4, NM_022561.2, and NM_022562.2.

The concentration of the growth hormone receptor agonist included in the differentiation medium is preferably 0.01 ng/mL to 1,000 ng/mL, and may be, for example, 0.1 ng/mL to 500 ng/mL, or for example, 1 ng/mL to 100 ng/mL.

Prolactin is a hormone that is secreted primarily from prolactin-secreting cells of the anterior lobe of the pituitary gland. It is considered that prolactin has a close genetic constitution and amino acid sequence to the growth hormone, one ancestral gene is overlapped, and the functions are differentiated.

Examples of the prolactin receptor agonist include a ligand having an agonistic action on the prolactin receptor. Examples of such a ligand include prolactin and a prolactin mimetic. Examples of the prolactin mimetic include an agonist antibody, an antibody fragment, and a peptide against the prolactin receptor. Examples of the NCBI accession number of the cDNA of human prolactin include NM_000948.6 and NM_001163558.3. In the production method of the present embodiment, a prolactin receptor agonist can be added to the medium instead of the growth hormone receptor agonist or together with the growth hormone receptor agonist.

The concentration of the prolactin receptor agonist included in the differentiation medium is preferably 0.01 ng/mL to 1,000 ng/mL, and may be, for example, 0.1 ng/mL to 500 ng/mL, or for example, 1 ng/mL to 100 ng/mL.

The glucocorticoid is also called a glucocorticoid, and is a type of adrenal cortex hormone produced in the zona fasciculata of the adrenal cortex. Examples of the glucocorticoid receptor agonist include a ligand having an agonistic action on the glucocorticoid receptor. Examples of such a ligand include a glucocorticoid and a glucocorticoid mimetic. Examples of the glucocorticoid mimetic include synthetic steroids such as prednisolone (CAS number: 50-24-8), dexamethasone (CAS number: 50-02-2), and betamethasone (CAS number: 378-44-9); and an agonist antibody, an antibody fragment, and a peptide against the glucocorticoid receptor. Examples of the glucocorticoid include cortisol (CAS number: 50-23-7), corticosterone (CAS number: 50-22-6), and cortisone (CAS number: 53-06-5).

The concentration of the glucocorticoid receptor agonist included in the differentiation medium is preferably 10 ng/mL to 1,000 ng/mL, and may be, for example, 10 ng/mL to 500 ng/mL, or for example, 10 ng/mL to 200 ng/mL.

The differentiation medium may include a growth hormone receptor agonist and a glucocorticoid receptor agonist, may include a prolactin receptor agonist and a glucocorticoid receptor agonist, or may include a growth hormone receptor agonist, a prolactin receptor agonist, and a glucocorticoid receptor agonist.

It is preferable that the differentiation medium further includes one or two growth factors selected from the group consisting of an epidermal growth factor (EGF), a hepatocyte growth factor (HGF), and a fibroblast growth factor (FGF).

EGF is a growth factor that activates an epidermal growth factor receptor (EGFR or ErbB1). Activated EGFR mainly activates the MAPK signaling pathway and also activates the PI3K signaling pathway and the Jak/stat signaling pathway.

The concentration of EGF included in the differentiation medium is preferably 10 ng/mL to 1,000 ng/mL, and may be, for example, 10 ng/mL to 500 ng/mL, or for example, 10 ng/mL to 200 ng/mL.

HGF is a growth factor that activates an Met receptor, and an activated Met receptor activates an HGF-Met signaling pathway. Activation of the HGF-Met signaling pathway promotes activation of the β-catenin pathway and promotes angiogenesis and metalloprotease production.

The concentration of HGF included in the differentiation medium is preferably 10 ng/mL to 1,000 ng/mL, and may be, for example, 10 ng/mL to 500 ng/mL, or for example, 10 ng/mL to 200 ng/mL.

FGF is preferably one that can bind to FGF receptor 2 (FGFR2) or FGF receptor 4 (FGFR4), and FGF is preferably FGF2, FGF4, FGF7, or FGF10, and particularly preferably FGF10.

The concentration of FGF included in the differentiation medium is preferably 20 ng/mL to 500 ng/mL, and may be, for example, 20 ng/mL to 300 ng/mL, or for example, 20 ng/mL to 150 ng/mL.

It is preferable that the differentiation medium further includes a transforming growth factor (TGF) β receptor antagonist.

TGFβ signal contributes to suppression of cell proliferation, differentiation of cells, induction of apoptosis, and the like. The TGFβ receptor antagonist is a substance that downregulates the TGFβ signal, and can also be referred to as a TGF-β signaling pathway inhibitor.

**In** the present specification, the TGFβ receptor antagonist refers to an inhibitor that inhibits the activation of an I type receptor or an II type receptor of a serine/threonine kinase type receptor, and is a TGFβ receptor antagonist accompanied by the inhibition of Smad2/3 phosphorylation.

Examples of the TGFβ receptor antagonist accompanied by the inhibition of phosphorylation of Smad2/3 include A83-01 (CAS number: 909910-43-6), SB-431542 (CAS number: 301836-41-9), SB-505124 (CAS number: 694433-59-5), SB-525334 (CAS number: 356559-20-1), LY364947 (CAS number: 396129-53-6), SD-208 (CAS number: 627536-09-8), and SJN2511 (CAS number: 446859-33-2), and among these, A83-01 is preferable.

The concentration of the TGFβ receptor antagonist included in the differentiation medium is preferably 0.05 µM to 50 µM, and may be, for example, 0.5 µM to 30 µM, or for example, 1 µM to 15 µM.

It is preferable that the differentiation medium further includes a γ-secretase inhibitor. Examples of the γ-secretase inhibitor include DAPT (CAS number: 208255-80-5) and dibenzazepine (CAS number: 256-96-2). These γ-secretase inhibitors function as an inhibitor of a Notch signal by inhibiting the Notch cleavage.

The concentration of the γ-secretase inhibitor included in the differentiation medium is preferably 0.05 µM to 50 µM, and may be, for example, 0.5 µM to 30 µM, or for example, 1 µM to 15 µM.

It is preferable that the differentiation medium further includes one or two or more selected from the group consisting of estradiol, dehydroepiandrosterone, and glucagon. Estradiol and dehydroepiandrosterone are types of sex steroid hormones that are biosynthesized in vivo using cholesterol as a raw material. Estradiol is a type of female hormone. Dehydroepiandrosterone is a type of male hormone. As described later in Examples, in a case where the differentiation medium includes these substances, the gluconeogenesis and the urea production in the liver organoid are promoted.

The concentration of estradiol included in the differentiation medium is preferably 10 nM to 50 µM, and may be, for example, 10 nM to 30 µM, or for example, 10 nM to 15 µM.

The concentration of dehydroepiandrosterone included in the differentiation medium is preferably 0.05 µM to 50 µM, and may be, for example, 0.5 µM to 30 µM, or for example, 1 µM to 15 µM.

The concentration of glucagon included in the differentiation medium is preferably 0.01 ng/mL to 1,000 ng/mL, and may be, for example, 0.1 ng/mL to 500 ng/mL, or for example, 1 ng/mL to 100 ng/mL.

Examples of other components that can be included in the differentiation medium include gastrin (or an appropriate substitute such as Leu15-gastrin I); antibacterial agents such as penicillin-based antibiotics, cephalosporin-based antibiotics, macrolide-based antibiotics, and tetracycline-based antibiotics; and cAMP inducers such as forskolin.

The differentiation medium can include a basic culture component such as an amino acid, a vitamin, an inorganic salt, and a carbon source such as glucose. As the basic culture component, a basic component included in a commercially available basic medium or a medium supplement can be used. Examples of the basic medium include a BME medium, a BGJb medium, a CMRL 1066 medium, a Glasgow MEM (GMEM) medium, an Improved MEM Zinc Option medium, an IMDM medium, a Medium 199 medium, an Eagle MEM medium, an αMEM medium, a DMEM medium, a F-12 medium, a DMEM/F12 medium, an IMDM/F12 medium, a Ham's medium, an RPMI 1640 medium, a serum mimetic medium, and a Fischer's medium; and a medium of a mixed medium thereof. Examples of the commercially available medium supplement include supplements containing insulin, such as B27 Supplement (Thermo Fisher Scientific) and N2 Supplement (Thermo Fisher Scientific).

The differentiation medium is generally prepared by adding the above-described components to a basic medium and, as necessary, adding a medium supplement.

Examples of the components that are preferably not included in the differentiation medium include components that upregulate Wnt signaling, such as Wnt proteins and R-spondin; IL-6 family cytokines such as interleukin-6, oncostatin, and a leukemia inhibitory factor (LIF); and bone morphogenetic protein (BMP) receptor antagonists that are accompanied by the phosphorylation inhibition of Smad1/5/9.

The production method of the present embodiment may further include, in the following order after the above-described step (a), a step (b) of fasting the metabolically activated liver organoid and a step (c) of culturing the metabolically activated liver organoid in a gluconeogenesis medium. Here, the gluconeogenesis medium is a medium including a gluconeogenesis substrate.

Gluconeogenesis refers to a means or pathway by which a human or a nonhuman animal produces glucose from substances other than saccharides such as pyruvic acid, lactic acid, gluconeogenic amino acids, propionic acid, and glycerol, using secretion of glucagon as a signal.

The inventors have found that by configuring the production method of the present embodiment to further include the step (b) and the step (c), gluconeogenesis and urea production of the metabolically activated liver organoid are enhanced.

Fasting refers to a state in which cells are maintained in a state where a carbon source such as glucose and glutamine is not supplied to the cells. Examples of the fasting method include a method of culturing cells in a fasting medium. Examples of the fasting medium include a medium that does not include or includes only a very small amount of a carbon source such as glucose and glutamine in the above-described basic culture component. It is preferable that the fasting medium does not include a component that adjusts the metabolism of sugar such as insulin.

In the production method of the present embodiment, it is preferable that the production component from the hepatocytes included in the culture supernatant of the differentiation culture is removed after culturing in the differentiation medium and before the fasting. Examples of the method include a method of washing the differentiation medium with Fasting Medium or the like, and then replacing the differentiation medium.

The gluconeogenesis medium is a medium including a gluconeogenesis substrate. Examples of the gluconeogenesis substrates include pyruvic acid, lactic acid, gluconeogenic amino acid, propionic acid, and glycerol. The gluconeogenesis medium includes one or two or more substrates among the gluconeogenesis substrates.

It is preferable that the gluconeogenesis medium further includes a glucagon receptor agonist. Glucagon is a hormone that is related to a cAMP circuit and increases a blood glucose level through production of glucose by glycogenolysis and gluconeogenesis in the liver.

Examples of the glucagon receptor agonist include a ligand having an agonistic action on the glucagon receptor. Examples of such a ligand include glucagon and a glucagon mimetic. Examples of the glucagon mimetic include an agonist antibody, an antibody fragment, and a peptide against the glucagon receptor. Examples of the NCBI accession number of cDNA of glucagon include NM_002054.5.

It is preferable that the gluconeogenesis medium further includes one or two selected from the group consisting of a growth hormone receptor agonist and a prolactin receptor agonist, and a glucocorticoid receptor agonist. The growth hormone receptor agonist, the prolactin receptor agonist, and the glucocorticoid receptor agonist are the same as those described above.

The gluconeogenesis medium can usually contain a basic culture component that does not include or includes only a very small amount of a carbon source such as glucose and glutamine in the above-described basic culture component, in the same manner as the fasting medium.

In the production method of the present embodiment, it is preferable that the primary hepatocytes or the hepatocyte-like cells are suspended in an extracellular matrix, the extracellular matrix is polymerized, and the medium is layered over the polymerized extracellular matrix to culture.

An extracellular matrix is a substance that serves as a scaffold for cells in cell culture. Examples of a component of the extracellular matrix include a component included in the basement membrane, and a glycoprotein present in the intercellular space. Examples of the component included in the basement membrane include type IV collagen, laminin, heparan sulfate proteoglycan, and entactin. Examples of the glycoprotein present in the intercellular space include collagen, laminin, entactin, fibronectin, fibrinogen, and heparin sulfate. As the component of the extracellular matrix, these components may be used alone or a combination of two or more kinds thereof may be used. The extracellular matrix preferably includes laminin, and more preferably includes laminin-111.

Examples of a commercially available product of the extracellular matrix include Matrigel (registered trademark), Cultrex (Bio-Techne Corporation), Geltrex (Thermo Fisher Scientific), EHS Gel Basement Membrane Matrix (FUJIFILM Wako Pure Chemical Corporation, Sigma), and Collagen I (Nitta Gelatin Inc.).

A hydrogel consisting of a synthetic polymer can also be used as a substance that serves as a scaffold for cells. Examples of a commercially available product of a hydrogel consisting of a synthetic polymer include Mebiol Gel (Mebiol Inc.), VitroGel (TheWell Bioscience), and GrowDex (UPM Biomedicals).

### [Composition]

In one embodiment, the present invention provides a composition including one or two selected from the group consisting of a growth hormone receptor agonist and a prolactin receptor agonist, and a glucocorticoid receptor agonist.

The composition of the present embodiment can be suitably used as a medium for producing a metabolically activated liver organoid. In the composition of the present embodiment, the growth hormone receptor agonist, the prolactin receptor agonist, and the glucocorticoid receptor agonist are the same as those described above.

### [Method for Evaluating Test Substance]

In one embodiment, the present invention provides a method for evaluating a test substance, the method including a step of bringing a test substance into contact with a metabolically activated liver organoid produced by the above-described production method; and a step of evaluating an effect of the test substance on the metabolically activated liver organoid.

The test substance is not particularly limited, and for example, natural compound libraries, synthetic compound libraries, existing drug libraries, or the like can be used.

As described later in Examples, the metabolically activated liver organoid produced by the above-described production method exhibits the same behavior as human hepatocytes. Therefore, according to the evaluation method of the present embodiment, the effect of the test substance on the hepatocytes in vivo in a human can be evaluated in vitro.

The effect of the test substance on the metabolically activated liver organoid can be evaluated by measuring, for example, gluconeogenesis, urea production, albumin production, bile acid production, and CYP activity.

### [Examples]

Hereinafter, the present embodiment will be described in more detail based on Examples. However, the present embodiment is not limited to these Examples.

### [Experimental Example 1]

### (Evaluation of Gluconeogenesis, Urea Production, Albumin Production, and Bile Acid Production of Primary Human Hepatocytes)

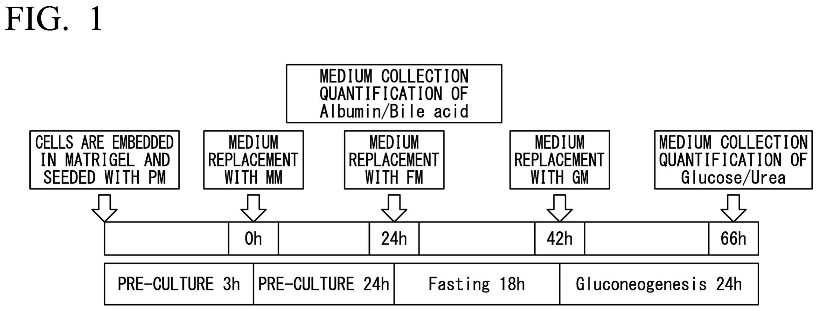

FIG. 1 is a view showing an evaluation procedure for gluconeogenesis, urea production, albumin production, and bile acid production of primary human hepatocytes (hereinafter sometimes referred to as "PHHs"). Frozen primary human hepatocytes were thawed and suspended in Primary Hepatocyte Thawing and Plating Medium (Thermo Fisher Scientific, hereinafter sometimes referred to as "PM medium"). The donor information of the primary human hepatocytes used is shown in Table 1 below.

**[Table 1]**

| | Manufacturer | Lot number | Floating/Attached | Sex | Age | Abbreviation |
|---|---|---|---|---|---|---|
| Donor 1 | BIOPREDIC | HEP187216 | Floating | Female | 39 | PHH1 |
| Donor 2 | BIOPREDIC | HEP187390-TA05 | Attached | Male | 53 | PHH2 |
| Donor 3 | Cytes | HuHeCPMI3D/4 | Attached | Female | 61 | PHH3 |
| Donor 4 | Cytes | HuHeCPMI/4- | Attached | Male | 63 | PHH4 |
| Donor 5 | BIOPREDIC | HEP187608 | Attached | Female | 39 | PHH5 |
| Donor 6 | Cytes | HuHeCS/6+ | Floating | Male | 51 | PHH6 |
| Donor 7 | Discovery Life Sciences | HH1136 | Attached | Male | 1.33 | PHH7 |
| Donor 8 | BiolVT | COR | Attached | Male | 30 | PHH8 |
| Donor 9 | BioIVT | MRW | Attached | Male | 0.92 | PHH9 |
| Donor 10 | BioIVT | OQV | Attached | Female | 37 | PHH10 |
| Donor 11 | Xenotech | HC2-19 | Attached | Female | 9 | PHH11 |

Subsequently, Matrigel (registered trademark, Corning) was added to the suspension of primary human hepatocytes and suspended. The suspension was seeded in a 24-well plate at 1 × 10⁴ cells/well. After gelation of Matrigel (registered trademark), a PM medium was added to and layered over the well at 500 µL/well, and incubation was performed for 3 hours in an environment of 37°C, 1 atm, and a CO₂ concentration of 5 v/v%.

Subsequently, the medium was replaced with Primary Hepatocyte Maintenance Medium (Thermo Fisher Scientific, hereinafter sometimes referred to as an "MM medium") at 500 µL/well, and incubation was performed for 24 hours in an environment of 37°C, 1 atm, and a CO₂ concentration of 5 v/v%. The supernatant of the medium after the incubation was collected, and albumin and bile acid included in the medium were quantified. The measurement results will be described later.

Human albumin (hereinafter sometimes referred to as "albumin") was measured according to a protocol of the manufacturer using Human Albumin ELISA Kit (Bethyl Laboratories, Inc., catalog number: E88-129).

The bile acid was measured using a system in which an electrospray ionization device Nexera X2 (Shimadzu Corporation) and a triple quadrupole type mass spectrometer LCMS-8040 (Shimadzu Corporation) were connected to an ultra-high performance liquid chromatograph. A mixed solution of a 0.1% aqueous acetic acid solution (A) and acetonitrile (B) was used as an eluent, and the flow rate was set to 0.3 mL/min. As the analysis column, Waters XBridge C18 (particle diameter: 3.5 µm, 2.1 × 50 mm, Waters) was used at 40°C. The gradient program of the eluent was set to 5% → 55% B (0 to 12 minutes), 55% → 95% B (12 to 12.5 minutes), 95% B (12.5 to 13.5 minutes), 95% → 5% B (13.5 to 13.51 minutes), and 5% B (13.51 to 17 minutes). The eluent of the column was subjected to electrospray ionization at an applied voltage of 4.5 kV and -3.5 kV in the positive and negative ionization modes, respectively, and then the bile acid was detected by mass spectrometry by multiple reaction monitoring. The ionization mode, the monitoring ion, and the collision energy of each analysis object are shown in Table 2 below.

**[Table 2]**

| Analysis object | Mode | m/z | | Collision Energy (eV) |
|---|---|---|---|---|
| | | Q1 | Q3 | |
| Cholic acid (CA) | negative ESI/MRM | 407.30 | 407.30 | 10 |
| Glycolic acid (GCA) | negative ESI/MRM | 464.30 | 74.00 | 41 |
| Taurocholic acid (TCA) | negative ESI/MRM | 514.30 | 80.00 | 55 |
| Chenodeoxycholic acid (CDCA) | negative ESI/MRM | 391.30 | 391.30 | 12 |
| Glycochenodeoxycholic acid (GCDCA) | negative ESI/MRM | 448.30 | 74.00 | 44 |
| Taurochenodeoxycholic acid (TCDCA) | negative ESI/MRM | 498.30 | 80.00 | 54 |
| Deoxycholic acid (DCA) | negative ESI/MRM | 391.30 | 391.30 | 12 |
| Glycodeoxycholic acid (GDCA) | negative ESI/MRM | 448.30 | 74.00 | 44 |

A four-fold volume of methanol was added to the culture supernatant to precipitate the proteins and the bile acids in the supernatant were measured by the system. The bile acid was measured by an absolute calibration curve method using a peak area. **In** addition, a calibration curve was created by an internal standard method using a known sample.

**In** addition, the primary human hepatocytes after the incubation were washed three times with Fasting Medium (hereinafter sometimes referred to as an "FM medium") having the composition shown in Table 3 below, the FM medium was added thereto at 500 µL/well, and incubation was performed for 18 hours in an environment of 37°C, 1 atm, and a CO₂ concentration of 5 v/v%.

**[Table 3]**

| Fasting Medium (FM) | | | |
|---|---|---|---|
| Component | Concentration | Manufacturer | Catalog Number |
| DMEM (glucose-free) | 1 x | Thermo Fisher Scientific | A1443001 |
| Penicillin-Streptomycin | 1 v/v% | Thermo Fisher Scientific | 15140122 |
| HEPES | 1 v/v% | Thermo Fisher Scientific | 15630130 |

Subsequently, the medium was replaced with a Gluconogenesis assay medium having the composition shown in Table 4 below (hereinafter sometimes referred to as a "GM medium"), and incubation was performed for 24 hours in an environment of 37°C, 1 atm, and a CO₂ concentration of 5 v/v%. The supernatant of the medium after the incubation was collected, and glucose and urea included in the medium were quantified. The measurement results will be described later.

**[Table 4]**

| Gluconeogenesis assay Medium (GM) | | | | |
|---|---|---|---|---|
| Component | | Concentration | Manufacturer | Catalog Number |
| DMEM (glucose-free) | | 1 x | Thermo Fisher Scientific | A1443001 |
| Gluconeogenesis substrates | Pyruvic Acid | Thermo Fisher Scientific | Thermo Fisher Scientific | 11360070 |
| | Lactic Acid | 1.5 mM | Sigma | L7022 |
| | Glycerol | 0.25 mM | FUJIFILM Wako Pure Chemical Corporation | 079-00614 |
| | GlutaMAX (alanyl-glutamine) | 2 mM | Thermo Fisher Scientific | 35050061 |
| Penicillin-streptomycin | | 1 v/v% | Thermo Fisher Scientific | 15140122 |
| HEPES | | 1 v/v% | Thermo Fisher Scientific | 15630130 |
| Growth hormone | | 10 ng/mL | PeproTech | 100-40 |
| Prolactin | | 10 ng/mL | PeproTech | 100-07 |
| Cortisol | | 100 ng/mL | Selleck | S1696 |
| Glucagon | | 200 ng/mL | Sigma | G2044 |

The glucose was measured according to a protocol of the manufacturer using a Glucose-Glo^{™} Assay (Promega, catalog number: J6022). A mixed solution of a 0.1% aqueous acetic acid solution (A) and acetonitrile (B) was used as an eluent, and the flow rate was set to 0.3 mL/min.

The urea was measured according to a protocol of the manufacturer using Quantichrom Urea Assay Kit (Bioassay Systems, catalog number: DIUR-100).

### [Experimental Example 2]

### (Evaluation 1 of Gluconeogenesis, Urea Production, Albumin Production, and Bile Acid Production in Liver Organoid)

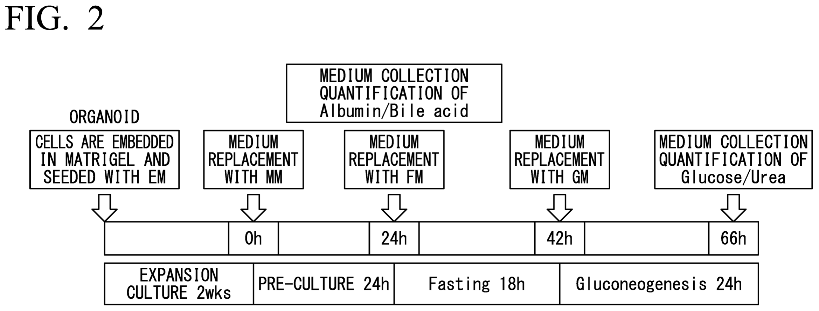

FIG. 2 is a view showing an evaluation procedure for gluconeogenesis, urea production, albumin production, and bile acid production of a liver organoid in the present Experimental Example.

### <<Production of Liver Organoid>>

The donor information of the primary human hepatocytes used is shown in Table 1. Frozen primary human hepatocytes were thawed and suspended in a medium obtained by adding HEPES, GLUTAMAX (Thermo Fisher Scientific), and penicillin/streptomycin (Thermo Fisher Scientific) to Advanced DMEM/F12 (Thermo Fisher Scientific), and centrifuged. The supernatant was removed and the cells were suspended in Advanced DMEM/F12. Subsequently, Matrigel (registered trademark) was added to the suspension of primary human hepatocytes and suspended. The suspension was seeded in a 24-well plate at 2 × 10⁴ cells/well. After gelation of Matrigel (registered trademark), expansion medium (hereinafter sometimes referred to as an "EM medium") having the composition shown in Table 5 below was added to and layered over the well at 500 µL/well, and incubation was performed for 14 days in an environment of 37°C, 1 atm, and a CO₂ concentration of 5 v/v%. The obtained liver organoids were subcultured a plurality of times.

**[Table 5]**

| Expansion Medium (EM) | | | |
|---|---|---|---|
| Component | Concentration | Manufacturer | Catalog Number |
| Advanced-DMEM/F12 | 1 x | Thermo Fisher Scientific | 12634010 |
| B27 Supplement | 1 x | Thermo Fisher Scientific | 17504001 |
| N-Acetyl-cysteine | 1 mM | Sigma-Aldrich | A9165-100G |
| Recombinant human FGF10 | 100 ng/mL | PeproTech | 100-26 |
| Recombinant human EGF | 50 ng/mL | PeproTech | AF-100-15 |
| Recombinant human HGF | 25 ng/mL | PeproTech | 100-39H |
| Human [Leu¹⁵]-gastrin I | 10 nM | Sigma-Aldrich | G9145 |
| A83-01 | 5 µm | Tocris Bioscience | 2939 |
| Forskolin | 10 µM | Cayman Chemical | 11018 |
| Wnt Conditioned medium | 20 v/v% | Homemade | - |
| R-spondin-1 conditioned medium | 5 v/v% | Homemade | - |
| Noggin | 25 ng/ml | PeproTech | 120-10C |
| Oncostatin M | 20 ng/ml | PeproTech | 300-10H |
| Penicillin/streptomycin | 1 v/v% | Thermo Fisher Scientific | 15140122 |
| HEPES | 1 v/v% | Thermo Fisher Scientific | 15630130 |
| GlutaMAX | 1 x | Thermo Fisher Scientific | 35050061 |

### <<Expansion Culture of Liver Organoid>>

The liver organoid obtained as described above was suspended in Advanced DMEM/F12 (Thermo Fisher Scientific). Matrigel (registered trademark) was added to the suspension and suspended. The suspension was seeded in a 24-well plate at 2 × 10⁴ cells/well. After gelation of Matrigel (registered trademark), an expansion medium having the composition shown in Table 5, in which Advanced-DMEM/F12 had been replaced with Plasma-like Medium, was added to and layered over the well at 500 µL/well, and incubation was performed for 2 weeks in an environment of 37°C, 1 atm, and a CO₂ concentration of 5 v/v%. The medium was replaced every 7 days. The plasma-like medium was prepared with reference to Cantor J. R., et al., Physiologic Medium Rewires Cellular Metabolism and Reveals Uric Acid as an Endogenous Inhibitor of UMP Synthase, Cell, 169 (2), 258-272, 2017.

### <<Evaluation of Gluconeogenesis, Urea Production, Albumin Production, and Bile Acid Production>>

The medium of the liver organoid was replaced with Primary Hepatocyte Maintenance Medium (Thermo Fisher Scientific, hereinafter sometimes referred to as an "MM medium") at 500 µL/well, and incubation was performed for 24 hours in an environment of 37°C, 1 atm, and a CO₂ concentration of 5 v/v%. The supernatant of the medium after the incubation was collected, and albumin and bile acid included in the medium were quantified. The measurement results will be described later.

In addition, the liver organoid after the incubation were washed three times with Fasting Medium (hereinafter sometimes referred to as an "FM medium") having the composition shown in Table 3, FM was added thereto at 500 µL/well, and incubation was performed for 18 hours in an environment of 37°C, 1 atm, and a CO₂ concentration of 5 v/v%.

Subsequently, the medium was replaced with a Gluconogenesis assay medium having the composition shown in Table 4 (hereinafter sometimes referred to as a "GM medium"), and incubation was performed for 24 hours in an environment of 37°C, 1 atm, and a CO₂ concentration of 5 v/v%. The supernatant of the medium after the incubation was collected, and glucose and urea included in the medium were quantified. The quantification of glucose and urea was performed in the same manner as in Experimental Example 1. The measurement results will be described later.

### [Experimental Example 3]

### (Evaluation 2 of Gluconeogenesis, Urea Production, Albumin Production, and Bile Acid Production in Liver Organoid)

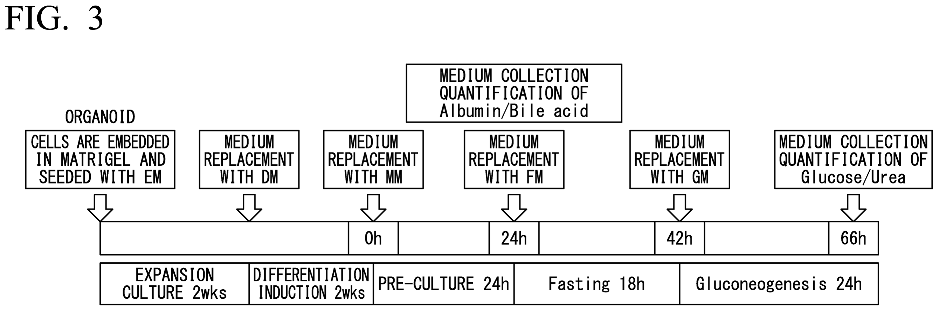

FIG. 3 is a view showing an evaluation procedure for gluconeogenesis, urea production, albumin production, and bile acid production of a liver organoid in the present Experimental Example. The present Experimental Example was different from Experimental Example 2 in that the liver organoid after the expansion culture was induced to differentiate.

### <<Differentiation Culture of Liver Organoid>>

The medium of the liver organoid, which had been produced in the same manner as in Experimental Example 2 and expansion-cultured for 2 weeks, was replaced with a differentiation medium (hereinafter sometimes referred to as a "DM medium") having the composition shown in Table 6 below at 500 µL/well, and incubated for 2 weeks in an environment of 37°C, 1 atm, and a CO₂ concentration of 5 v/v%. The medium was replaced every 7 days.

**[Table 6]**

| Differentiation Medium (DM) | | | |
|---|---|---|---|
| Component | Concentration | Manufacturer | Catalog Number |
| Plasma-like Medium | 1 x | Homemade | - |
| B27 Supplement | 1 x | Thermo Fisher Scientific | 17504001 |
| N-Acetyl-cysteine | 1 mM | Sigma-Aldrich | A9165-100G |
| Recombinant human FGF10 | 100 ng/ml | PeproTech | 100-26 |
| Recombinant human EGF | 50 ng/ml | PeproTech | AF-100-15 |
| Recombinant human HGF | 25 ng/ml | PeproTech | 100-39H |
| Human [Leu^15]-gastrin I | 10 nM | Sigma-Aldrich | G9145 |
| A83-01 | 5 µM | Tocris Bioscience | 2939 |
| Forskolin | 10 µM | Cayman Chemical | 11018 |
| Penicillin/streptomycin | 1 v/v% | Thermo Fisher Scientific | 15140122 |
| HEPES | 1 v/v% | Thermo Fisher Scientific | 15630130 |
| GlutaMAX | 1 x | Thermo Fisher Scientific | 35050061 |
| DAPT | 10 µM | Selleck | S2215 |
| Growth hormone | 10 ng/ml | PeproTech | 100-40 |
| Prolactin | 10 ng/ml | PeproTech | 100-07 |
| Cortisol | 100 ng/ml | Selleck | S1696 |

### <<Evaluation of Gluconeogenesis, Urea Production, Albumin Production, and Bile Acid Production>>

Subsequently, gluconeogenesis, urea production, albumin production, and bile acid production of the liver organoid were evaluated in the same manner as in Experimental Example 2. The results of the amount of glucose produced and the amount of urea produced will be described later.

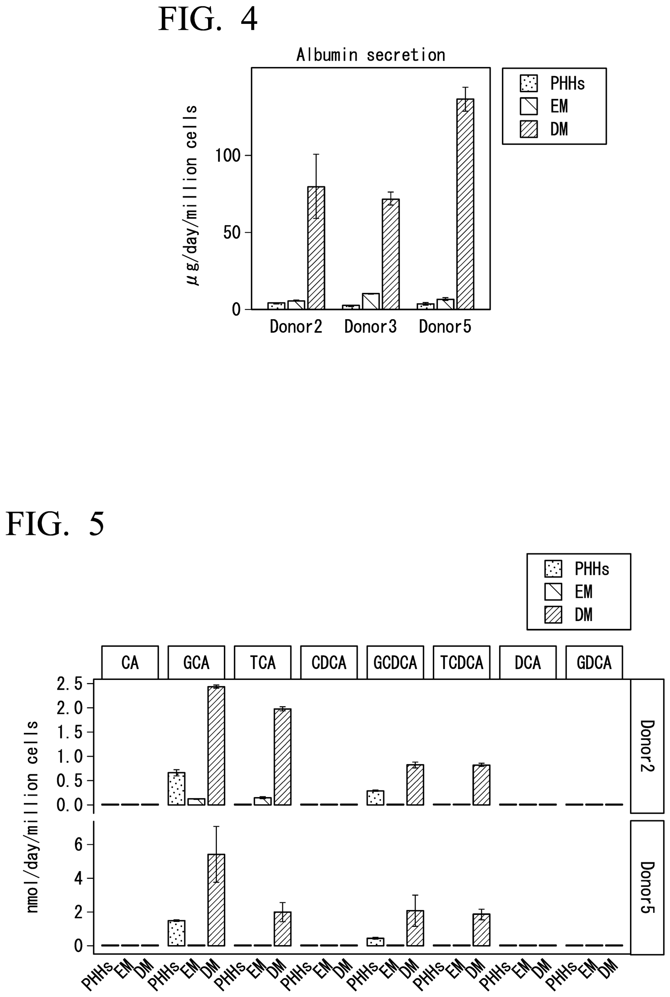

FIG. 4 is a graph showing the amount of albumin produced, as measured in Experimental Examples 1 to 3. In addition, estimated values of the amount of albumin, urea, and glucose produced by human hepatocytes are shown in Table 7 below. In FIG. 4, "PHHs" indicates the measurement results of the primary human hepatocytes in Experimental Example 1, "EM" indicates the measurement results of the liver organoids in Experimental Example 2, and "DM" indicates the measurement results of the liver organoids in Experimental Example 3.

**[Table 7]**

| | Estimated production amount |
|---|---|
| Albumin (µg/day/10⁶ hepatocytes) | 37 to 105 |
| Urea (µg/day/10⁶ hepatocytes) | 56 to 159 |
| Glucose (nmol/hour/10⁶ hepatocytes) | 100 (Fasting) |

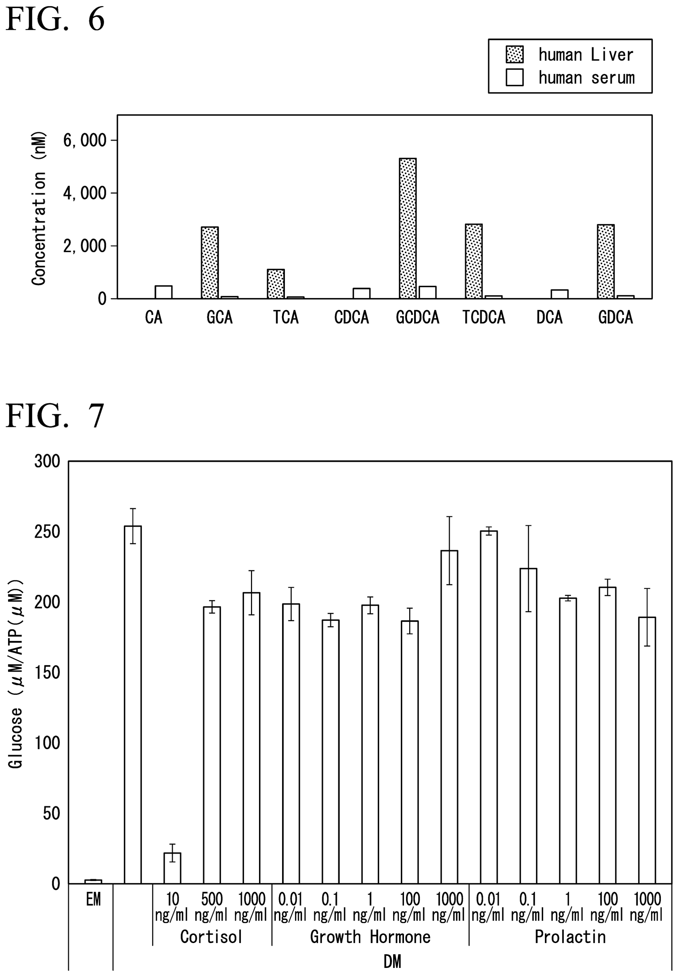

FIG. 5 is a graph showing the amount of bile acid produced, as measured in Experimental Examples 1 to 3. In addition, FIG. 6 is a graph showing the concentrations of bile acids in general human liver tissue and human serum. It is said that the daily bile acid synthesis amount in the human liver (2.5 × 10¹¹ cells) is 200 to 500 mg. In FIG. 5, "PHHs" indicates measurement results of primary human hepatocytes in Experimental Example 1, "EM" indicates the measurement results of liver organoids in Experimental Example 2, and "DM" indicates the measurement results of liver organoids in Experimental Example 3. In FIGS. 5 and 6, "CA" indicates cholic acid, "GCA" indicates glycolic acid, "TCA" indicates taurocholic acid, "CDCA" indicates chenodeoxycholic acid, "GCDCA" indicates glycochenodeoxycholic acid, "TCDCA" indicates taurochenodeoxycholic acid, "DCA" indicates deoxycholic acid, and "GDCA" indicates glycodeoxycholic acid.

### [Experimental Example 4]

### (Examination on Composition of Differentiation Medium)

The gluconeogenesis of the liver organoid was evaluated in the same manner as in Experimental Example 3, except that the composition of the differentiation medium was changed. Under the conditions shown in Table 8 below, a medium obtained by removing the growth hormone, the prolactin, and/or the cortisol from the differentiation medium having a composition shown in Table 6 and adding glucagon as a medium was used. In a case where glucagon was added, the concentration of glucagon was 200 ng/mL. In addition, the donor of the primary human hepatocyte used for the production of the liver organoid was a donor 1. The quantification value of glucose are shown in Table 8 below.

**[Table 8]**

| No. | Growth hormone prolactin | Cortisol | Glucagon | Glucose (nmol/10⁶ cells/hr) |
|---|---|---|---|---|
| 1 | + | + | + | 345.5 |
| 2 | + | + | - | 537.94 |
| 3 | + | - | - | 57.02 |
| 4 | - | - | - | 38.07 |

As a result, it was revealed that gluconeogenesis is most improved under the conditions in which a growth hormone, prolactin, and cortisol are added to the differentiation medium. In addition, it was revealed that the gluconeogenesis is improved under conditions in which a growth hormone, prolactin, cortisol, and glucagon are added to the differentiation medium.

### [Experimental Example 5]

### (Examination on Optimum Concentration of Composition of Differentiation Medium)

The gluconeogenesis of the liver organoid was evaluated in the same manner as in Experimental Example 3, except that the composition of the differentiation medium was changed. The addition concentrations of cortisol, the growth hormone, and prolactin were changed to the conditions shown in Table 9 below from the differentiation medium having the composition shown in Table 6, and used for culture. In addition, the donor of the primary human hepatocyte used for the production of the liver organoid was a donor 9.

**[Table 9]**

| Condition | Cortisol (ng/mL) | Growth hormone (ng/mL) | Prolactin (ng/mL) |
|---|---|---|---|
| 1 | 10 | 10 | 10 |
| 2 | 500 | 10 | 10 |
| 3 | 1,000 | 10 | 10 |
| 4 | 100 | 0.01 | 10 |
| 5 | 100 | 0.1 | 10 |
| 6 | 100 | 1 | 10 |
| 7 | 100 | 100 | 10 |
| 8 | 100 | 1,000 | 10 |
| 9 | 100 | 10 | 0.01 |
| 10 | 100 | 10 | 0.1 |
| 11 | 100 | 10 | 1 |
| 12 | 100 | 10 | 100 |
| 13 | 100 | 10 | 1,000 |

FIG. 7 is a graph showing the quantification results of glucose. The numerical values shown in FIG. 7 are numerical values corrected by the amount of ATP measured by 3D-Cell Titer Glo assay. In FIG. 7, "EM" indicates an expansion medium and "DM" indicates a differentiation medium. The measurement by 3D-Cell Titer Glo assay was performed according to a protocol of the manufacturer using CellTiter-Glo (R) 3D Cell Viability Assay (Promega Corporation, G9682).

Subsequently, the gluconeogenesis of the liver organoid was evaluated in the same manner as in Experimental Example 3, except that a differentiation medium obtained by changing the addition concentrations of cortisol, the growth hormone, and prolactin according to the conditions shown in Table 10 below from the differentiation medium having the composition shown in Table 6 was used. In addition, the donor of the primary human hepatocyte used for the production of the liver organoid was a donor 9.

**[Table 10]**

| Condition | Cortisol (ng/mL) | Growth hormone (ng/mL) | Prolactin (ng/mL) |
|---|---|---|---|
| 1 | 0 | 0 | 0 |
| 2 | 10 | 0 | 0 |
| 3 | 100 | 0 | 0 |
| 4 | 500 | 0 | 0 |
| 5 | 1,000 | 0 | 0 |
| 6 | 0 | 0. 1 | 0 |
| 7 | 0 | 10 | 0 |
| 8 | 0 | 100 | 0 |
| 9 | 0 | 1,000 | 0 |
| 10 | 0 | 0 | 0.1 |
| 11 | 0 | 0 | 10 |
| 12 | 0 | 0 | 100 |
| 13 | 0 | 0 | 1,000 |

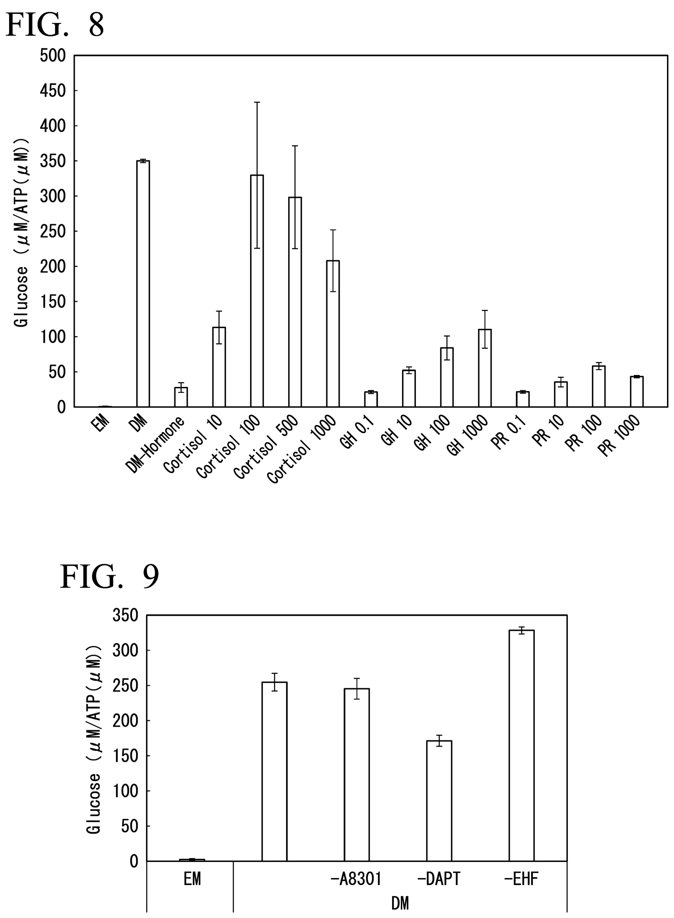

FIG. 8 is a graph showing the quantification results of glucose. The numerical values shown in FIG. 8 are numerical values corrected by the amount of ATP measured by 3D-Cell Titer Glo assay. In FIG. 8, "EM" indicates an expansion medium and "DM" indicates a differentiation medium. In addition, "GH" indicates a growth hormone, "PR" indicates prolactin, and "-Hormone" indicates that cortisol, a growth hormone, and prolactin are not included.

As a result of FIGS. 7 and 8, it was revealed that the gluconeogenesis of the liver organoid is improved in a manner dependent on the concentrations of added factors by using a differentiation medium including at least one of a growth hormone, prolactin, and cortisol. Furthermore, it was revealed that the gluconeogenesis by the liver organoid is improved by using a differentiation medium including growth hormone, prolactin, and cortisol, as compared with using a differentiation medium including at least one of a growth hormone, prolactin, and cortisol.

### [Experimental Example 6]

### (Examination on Composition of Differentiation Medium)

The gluconeogenesis of the liver organoid was evaluated in the same manner as in Experimental Example 3, except that the composition of the differentiation medium was changed. A medium obtained by removing A83-01, a medium obtained by removing DAPT, or a medium obtained by removing recombinant human FGF10, recombinant human EGF, and recombinant human HGF, each from the differentiation medium having the composition shown in Table 6, was used. In addition, the donor of the primary human hepatocyte used for the production of the liver organoid was a donor 9.

The quantification results of glucose are shown in FIG. 9. The numerical values shown in FIG. 9 are numerical values corrected by the amount of ATP measured by the 3D-Cell Titer Glo assay, as in Experimental Example 5. In FIG. 9, "EM" indicates an expansion medium and "DM" indicates a differentiation medium. In addition, "-A83-01" indicates a medium from which A83-01 had been removed, "-DAPT" indicates a medium from which DAPT had been removed, and "-EHF" indicates a medium from which recombinant human FGF10, recombinant human EGF, and recombinant human HGF had been removed.

As a result, it was revealed that gluconeogenesis by the liver organoid is recognized even in a case of using a medium obtained by removing A83-01, a medium obtained by removing DAPT, or a medium obtained by removing recombinant human FGF10, recombinant human EGF, and recombinant human HGF, each from the differentiation medium having the composition shown in Table 6.

### [Experimental Example 7]

### (Examination on Differentiation Culture Period)

The gluconeogenesis and the urea production in the liver organoid were evaluated in the same manner as in Experimental Example 2, except that the number of days of incubation in the expansion medium was changed. In addition, the gluconeogenesis and the urea production in the liver organoid were evaluated in the same manner as in Experimental Example 3, except that the number of days of incubation in the differentiation medium was changed.

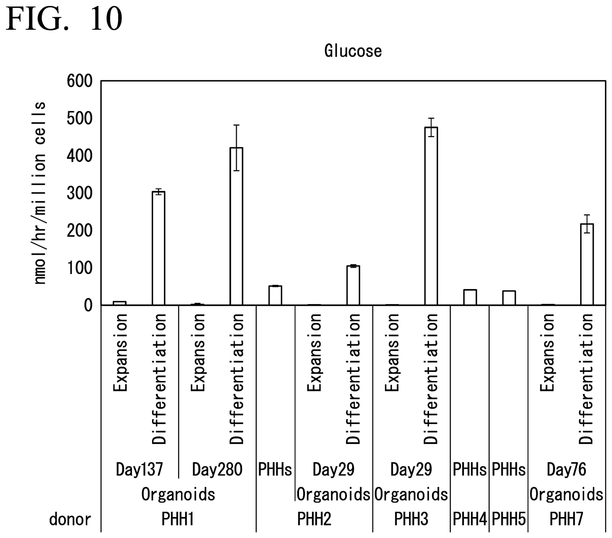

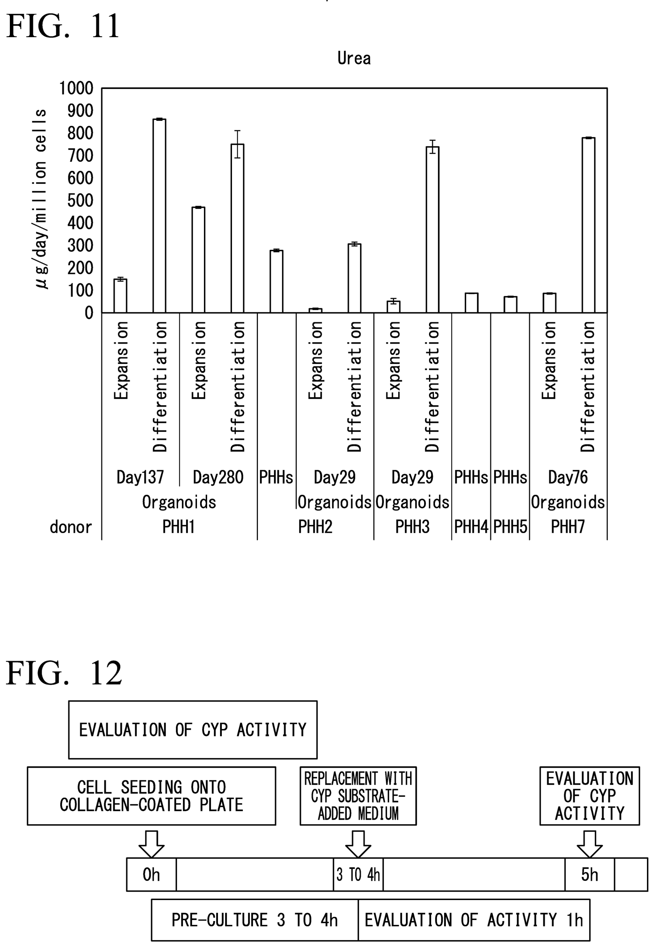

FIG. 10 is a graph showing the measurement results of glucose. In addition, FIG. 11 is a graph showing the measurement results of urea. In FIGS. 10 and 11, "Expansion" indicates the results of not culturing in a differentiation medium and "Differentiation" indicates the results of culturing in a differentiation medium. In addition, the number of days indicates the number of days of culturing in the expansion medium in a case where the culture is not carried out in the differentiation medium, and the total number of days of culturing in the expansion medium and the differentiation medium in a case where the culture is carried out in the differentiation medium. "Day 29" indicates the results of the liver organoid of Experimental Example 2 or 3.

As a result, it was revealed that the liver organoid cultured in the differentiation medium has a high amount of glucose produced.

### [Experimental Example 8]

### (Measurement of CYP Activity)

The CYP activities of the primary human hepatocyte, the liver organoid obtained by culturing in the expansion medium (EM medium) in Experimental Example 2, and the liver organoid obtained by culturing in the differentiation medium (DM medium) in Experimental Example 3 were measured.

A procedure for measuring the CYP activity is shown in FIG. 12. Frozen primary human hepatocytes were thawed and incubated in a PM medium in the same manner as in Experimental Example 1. Subsequently, the medium was replaced with an MM medium and the cells were suspended. Matrigel (registered trademark, Corning) was added to the suspension and suspended. The suspension was seeded on a 96-well collagen-coated plate (IWAKI, catalog number: #4860-010) at 1 × 10⁵ cells/well and allowed to stand for 3 hours.

The MM medium was replaced with a medium obtained by adding the MM medium and a cocktail substrate for CYP analysis to a William's E medium, and allowed to stand for 1 hour. A mixture of substrates shown in Table 11 below was used as a cocktail substrate for CYP analysis.

**[Table 11]**

| Target CYP | Substrate |
|---|---|
| CYP1A2 | 20 µM Phenacetin |
| CYP2A6 | 2 µM Coumarin |
| CYP2B6 | 5 µM Bupropion |
| CYP2C8 | 0.1 µM Amodiaquine |
| CYP2C9 | 1 µM Diclofenac |
| CYP2C19 | 40 µM S-Mephenytoin |
| CYP2D6 | 5 µM Bufuralol |
| CYP3A4 | 2 µM Midazolam and 10 µM Testosterone |

After allowing the mixture to stand for 1 hour, an internal standard substance solution (a 1:1 mixed solution of acetonitrile and methanol, including 0.2 µM quinidine, 0.2 µM ibuprofen, and 0.1% formic acid) in an amount equal to that of the medium was added to the well to remove the proteins, and the supernatant was subjected to LC/MS to measure the CYP metabolites. The quantification of the metabolite was performed by an internal standard calibration curve method using a peak area. Quinidine included in the internal standard substance solution was used as an internal standard in the positive mode, and ibuprofen was used as an internal standard in the negative mode.

LC/MS was measured using a system in which an electrospray ionization device Nexera X2 (Shimadzu Corporation) and a triple quadrupole type mass spectrometer LCMS-8040 (Shimadzu Corporation) were connected to an ultra-high performance liquid chromatograph. A mixed solution of a 0.1% aqueous acetic acid solution (A) and acetonitrile (B) was used as an eluent, and the flow rate was set to 0.3 mL/min. As an analysis column, YMC Triart C18 (particle diameter: 3 µm, 2.0 × 50 mm, YMC) was used at 40°C. The gradient program of the eluent was set to 5% → 95% B (0 to 4 minutes), 95% B (4 to 5 minutes), 95% → 5% B (5 to 5.01 minutes), and 5% B (5.01 to 8 minutes). The eluent of the column was subjected to electrospray ionization at an applied voltage of 4.5 kV and -3.5 kV in the positive and negative ionization modes, respectively, and then the CYP metabolite was detected by mass spectrometry by multiple reaction monitoring. The ionization mode, the monitoring ion, and the collision energy of each analysis object are shown in Table 12 below.

**[Table 12]**

| Analysis object | Mode | m/z | | Collision Energy (eV) |
|---|---|---|---|---|
| | | Q1 | Q3 | |
| Acetaminophen | positive ESI/MRM | 152.00 | 110.00 | -14 |
| 7-Hydroxycoumarin | negative ESI/MRM | 161.02 | 132.95 | 22 |
| 7-Hydroxycoumarin Glucuronide | negative ESI/MRM | 337.05 | 161.00 | 25 |
| 7-Hydroxycoumarin Sulfate | negative ESI/MRM | 240.98 | 161.00 | 18 |
| Hydroxybupropion | positive ESI/MRM | 256.11 | 238.10 | -12 |
| N-Desethylamodiaquine | positive ESI/MRM | 328.12 | 283.00 | -19 |
| 4'-Hydroxydiclofenac | positive ESI/MRM | 312.02 | 230.10 | -37 |
| 4'-Hydroxymephenytoin | negative ESI/MRM | 233.09 | 190.00 | 14 |
| 1'-Hydroxybufuralol | positive ESI/MRM | 278.18 | 186.10 | -19 |
| 1'-Hydroxymidazolam | positive ESI/MRM | 342.08 | 203.00 | -29 |
| 6β-Hydroxytestosterone | positive ESI/MRM | 305.00 | 269.00 | -15 |
| Quinidine | positive ESI/MRM | 325.19 | 81.10 | -36 |
| Ibuprofen | negative ESI/MRM | 205.12 | 161.10 | 9 |

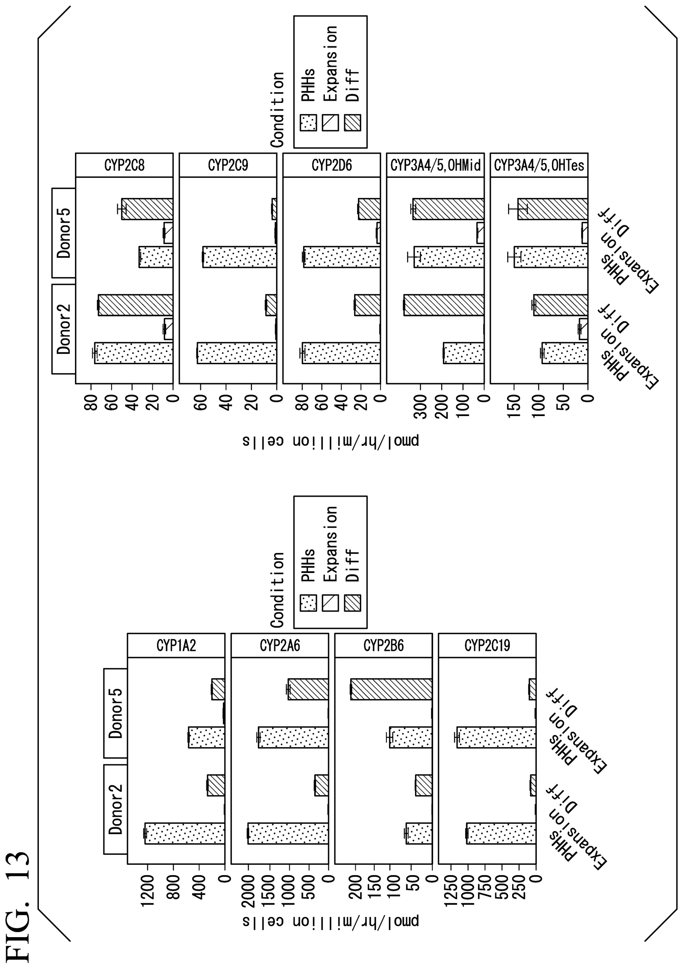

FIG. 13 is a graph showing the measurement results of the CYP metabolites. A large amount of CYP metabolite produced indicates that the CYP activity is high. In FIG. 13, "PHHs" indicates the results of a primary human hepatocyte, "Expansion" indicates the results of a liver organoid obtained by culturing in the expansion medium (EM medium), and "Diff" indicates the results of a liver organoid obtained by culturing in the differentiation medium (DM medium).

As a result, it was revealed that the liver organoid obtained by culturing in the differentiation medium had a significantly higher CYP activity than the liver organoid not cultured in the differentiation medium (the liver organoid obtained by culturing in the expansion medium).

### [Experimental Example 9]

### [CYP Induction Test]

The CYP activities of the primary human hepatocyte and the liver organoid obtained by culturing in the differentiation medium (DM medium) in Experimental Example 3 were induced and analyzed.

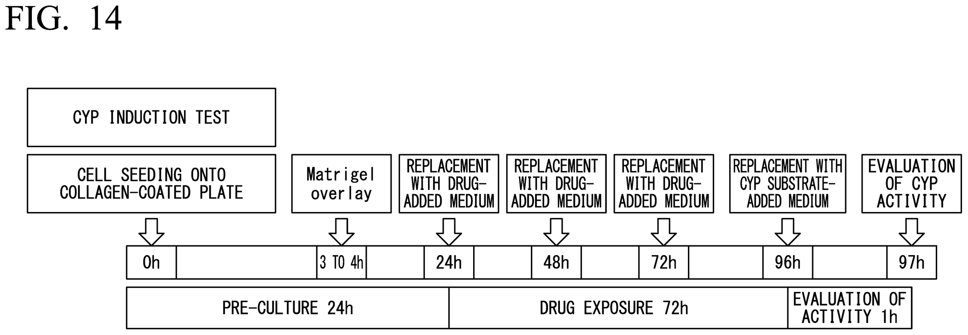

A procedure of the present Experimental Example is shown in FIG. 14. Frozen primary human hepatocytes were thawed and incubated in a PM medium in the same manner as in Experimental Example 1. Subsequently, the medium was replaced with an MM medium and the cells were suspended. 250 µg/mL of Matrigel (registered trademark, Corning) was added to the suspension and suspended. The suspension was seeded on a 96-well collagen-coated plate (IWAKI, catalog number: #4860-010) at 1 × 10⁵ cells/well and allowed to stand for 3 hours. Subsequently, the medium was replaced with an MM medium including 250 µg/mL of Matrigel. Subsequently, after 24 hours, the medium was replaced with an MM medium to which a drug shown in Table 13 below had been added.

Furthermore, rifampicin is a drug known to induce CYP3A4. Moreover, phenobarbital is a drug known to induce CYP2B6 and CYP3A4. In addition, omeprazole is a drug known to induce CYP1A2. The present Experimental Example can also be said to evaluate drug interaction or compatibility of medications and substances.

**[Table 13]**

| | Substance |
|---|---|
| Control group | 0.1% Dimethyl sulfoxide (DMSO) |
| Rifampicin group | 10 µM Rifampicin |
| Phenobarbital group | 1 mM Phenobarbital |
| Omeprazole group | 50 µM Omeprazole |

Thereafter, the medium was replaced with a medium to which each drug had been added every other day, and the cells were exposed to each drug for 3 days. Then, the CYP metabolite was measured in the same manner as in Experimental Example 9.

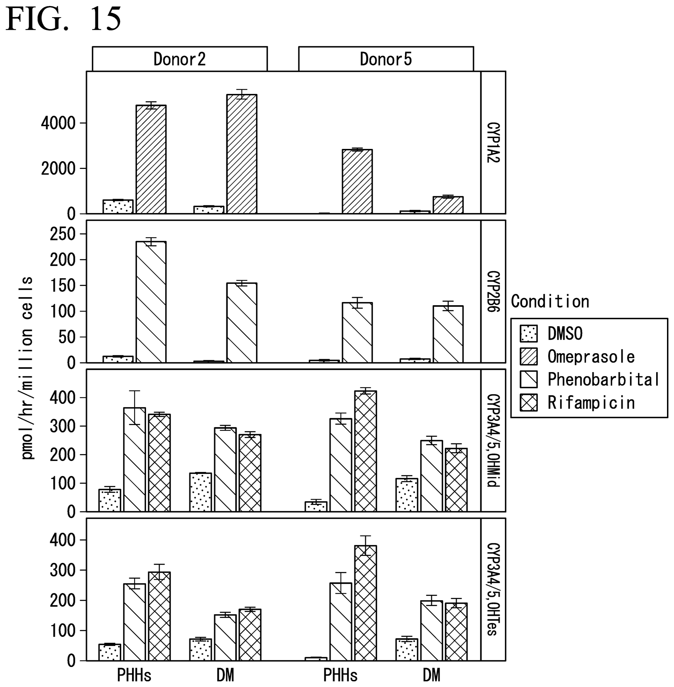

FIG. 15 is a graph showing the measurement results of the CYP metabolite. A large amount of CYP metabolite produced indicates that the CYP activity is high. In FIG. 15, "PHHs" indicates the results of a primary human hepatocyte, and "DM" indicates the results of a liver organoid obtained by culturing in the differentiation medium (DM medium).

As a result, it was confirmed that the addition of each drug to the medium resulted in the induction of CYP activity. In addition, it has been found that the liver organoid obtained by culturing in the DM medium exhibits behavior similar to that of the human hepatocyte.

### [Experimental Example 10]

### (Evaluation of Expression of Gluconeogenesis, Urea Production, Albumin Production, Bile Acid Production-Related Gene, CYP Gene, and Transporter-Related Gene)

RNA-seq analysis was performed on the primary human hepatocyte, the liver organoid obtained by culturing in the expansion medium (EM medium) in Experimental Example 2, and the liver organoid obtained by culturing in the differentiation medium (DM medium) in Experimental Example 3. The expression of a gluconeogenesisrelated gene, a urea production-related gene, an albumin production-related gene, a bile acid production-related gene, a CYP gene, and a transporter-related gene was evaluated.

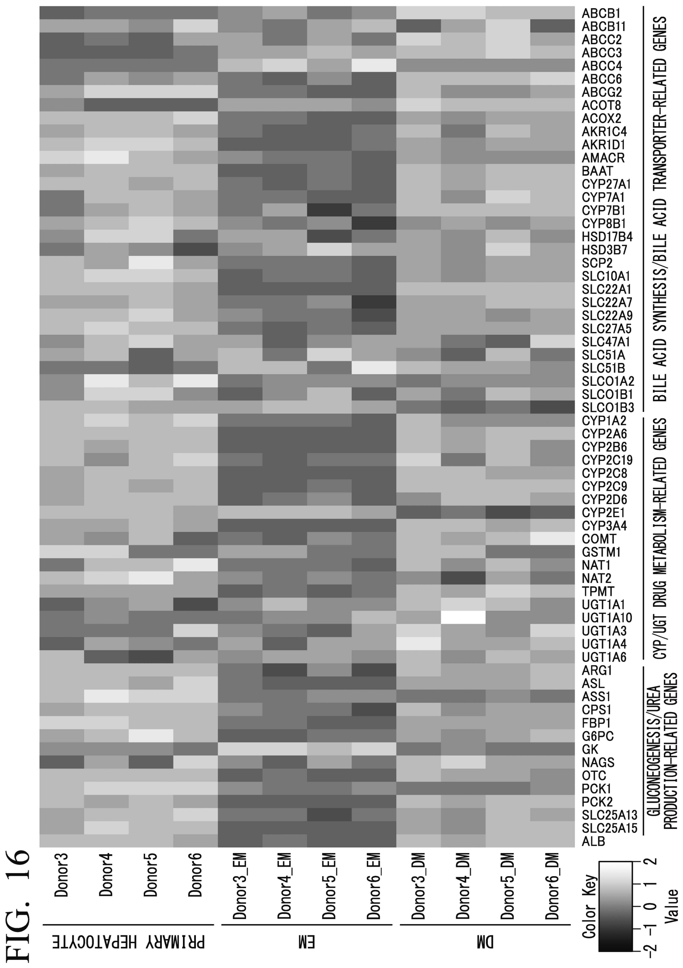

FIG. 16 is a heat map showing the expression of each gene. In FIG. 16, "EM" indicates the results of a liver organoid obtained by culturing in the expansion medium (EM medium), and "DM" indicates the results of a liver organoid obtained by culturing in the differentiation medium (DM medium). As a result, it was revealed that in the liver organoid obtained by culturing in the DM medium, the expression levels of these genes were close to the expression levels in the primary human hepatocytes.

### [Experimental Example 11]

### (Effect of Metformin)

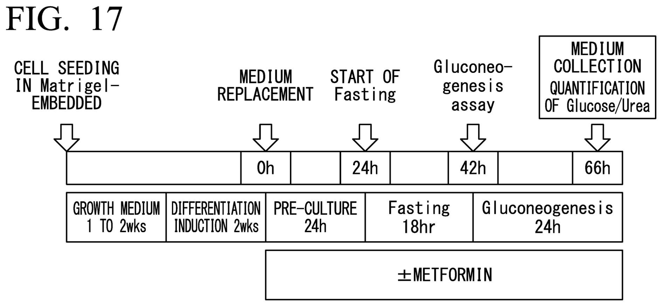

It is known that metformin has an effect of suppressing gluconeogenesis of human hepatocytes. Therefore, the gluconeogenesis and the urea production were evaluated in the same manner as in Experimental Example 3, except that metformin was added to the MM medium, the FM medium, and the GM medium to have a concentration of 1 mM or 10 mM. In addition, for the purpose of comparison, a group in which liver organoids had been produced in the same manner as in Experimental Example 2 and a group in which metformin had not been added were also prepared. FIG. 17 is a view showing a procedure of the present Experimental Example.

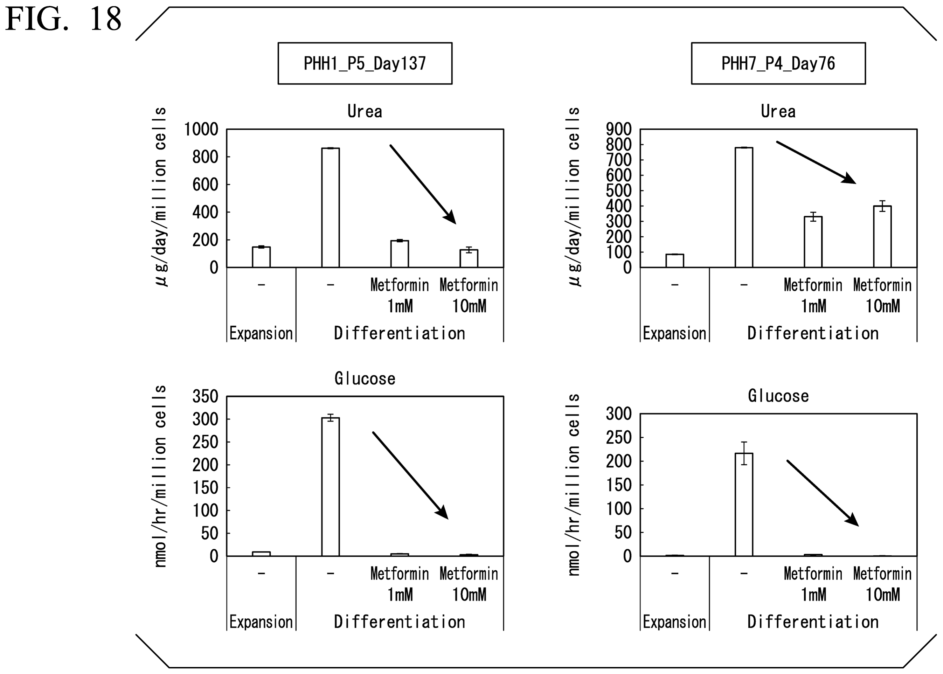

FIG. 18 is a graph showing the measurement results of urea and glucose. In FIG. 18, "Expansion" indicates the results of not culturing in a differentiation medium (DM medium), and "Differentiation" indicates the results of culturing in a DM medium.

As a result, it was shown that the addition of metformin to the medium suppressed gluconeogenesis and urea production in the liver organoid. As a result, it was revealed that the liver organoid obtained by culturing in the DM medium exhibited the same behavior as the human hepatocyte, as in Experimental Example 3.

### [Experimental Example 12]

### (Examination on Differentiation Culture Conditions)

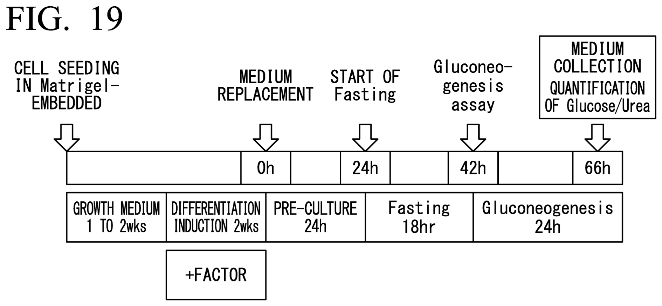

Liver organoids were produced, and the gluconeogenesis and the urea production were evaluated in the same manner as in Experimental Example 3, except that a factor shown in Table 14 below was added to the differentiation medium (DM medium). FIG. 19 is a view showing a procedure of the present Experimental Example.

**[Table 14]**

| Factors |
|---|
| 100 nM Estradiol |
| 1 µM Estradiol |
| 10 µM Dehydroepiandrosterone (DHEA) |
| 200 ng/mL Glucagon |
| 100 nM Estradiol, 10 µM DHEA, 200 ng/mL Glucagon |

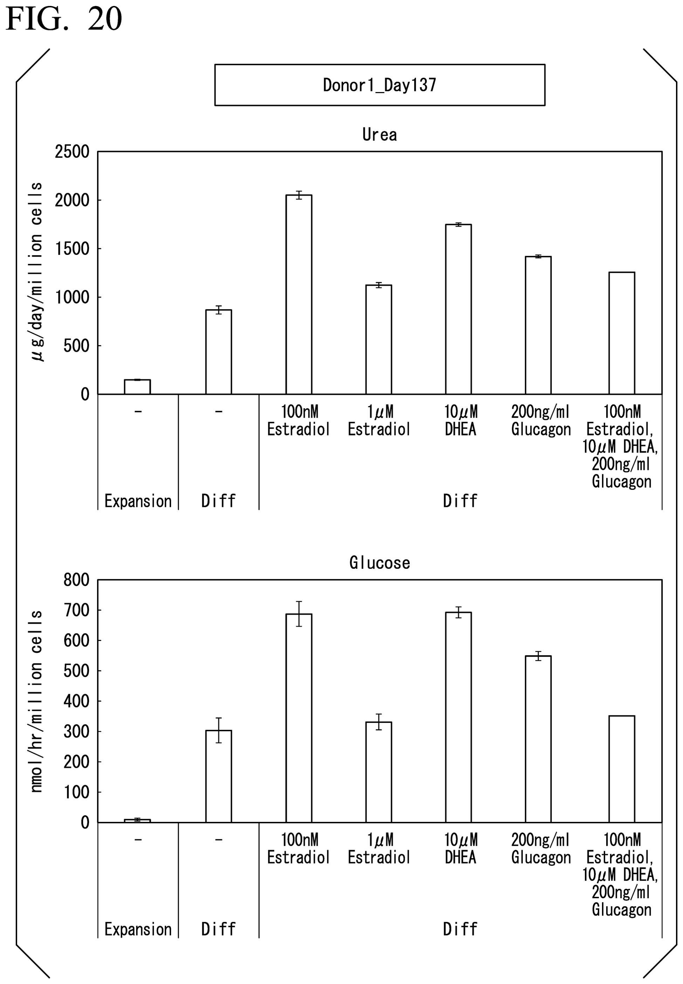

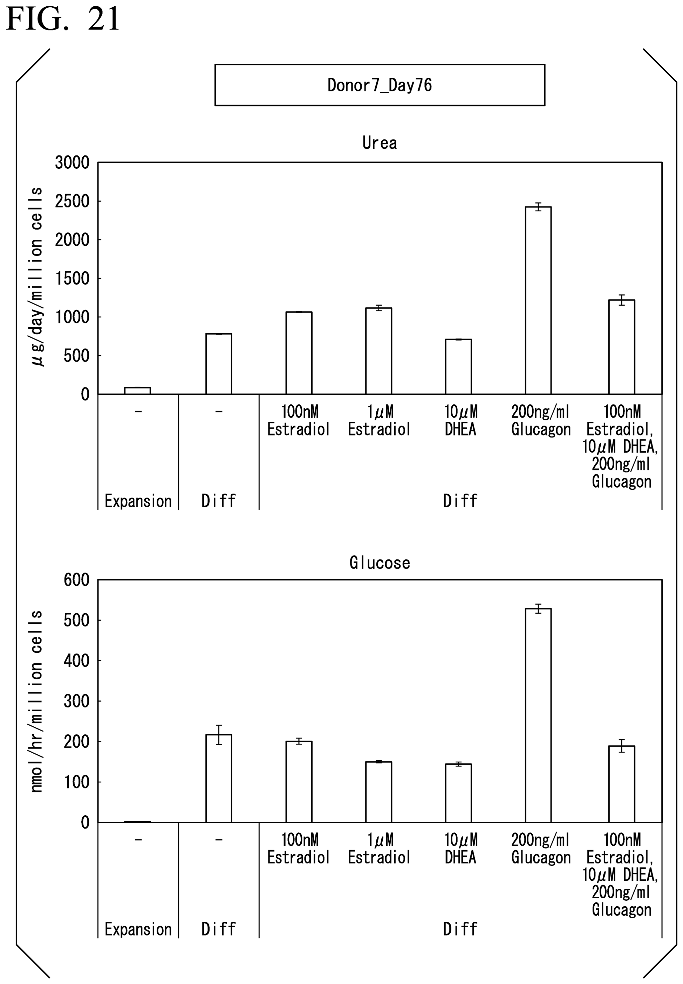

FIGS. 20 and 21 are graphs showing the measurement results of urea and glucose. In FIGS. 20 and 21, "Expansion" indicates the results of the liver organoid that was not cultured in the differentiation medium (DM medium), "Diff" indicates the results of the liver organoid that was cultured in the DM medium, and "-" indicates the results of the liver organoid in which a factor shown in Table 14 was not added.

As a result, it was shown that in a case where a factor shown in Table 14 is added to the DM medium, the gluconeogenesis and the urea production in the liver organoid are promoted.

### [Experimental Example 13]

### (Evaluation of Glycogen after Fasting)

Liver organoids were induced to differentiate in the same manner as in Experimental Example 3, except that the composition of the FM medium was changed and the incubation time after the addition of the FM medium was carried out for 18 hours or 24 hours. After culturing in the FM medium, intracellular glycogen was quantified.

The quantification of glycogen was performed according to a protocol of the manufacturer using Glycogen-Glo^{™} Assay (Promega Corporation, J5052). An FM medium having a composition shown in Table 3, an FM medium to which 200 ng/mL glucagon had been added, a mixed solution (also referred to as "Hormone Mix") of 10 ng/mL growth hormone, 10 ng/mL prolactin, and 100 ng/mL cortisol, and an FM medium to which 200 ng/mL glucagon had been added were used. In addition, the donor of the primary human hepatocyte used for the production of the liver organoid was a donor 8.

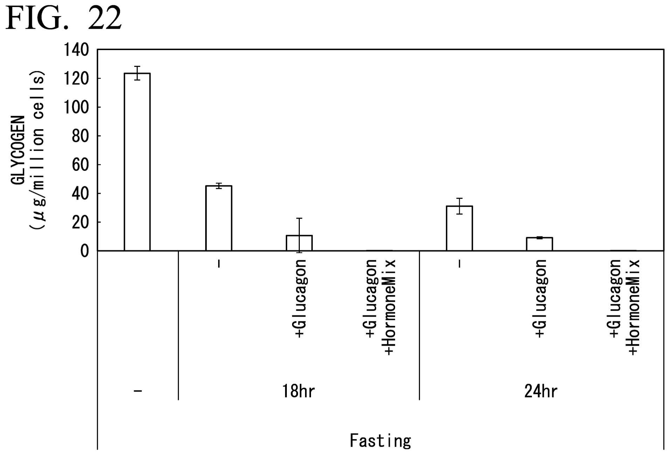

The quantification value of glycogen is shown in FIG. 22. As a result, it was shown that the glycogen was reduced from the liver organoid due to fasting.

### [Experimental Example 14]

### (Examination 1 on Optimum Concentration of Composition of Gluconeogenesis Medium)

A GM medium obtained by removing glucagon and cortisol from the gluconeogenesis medium (GM medium) having the composition shown in Table 4, and further adding the components shown in Table 15 was prepared. Liver organoids were produced in the same manner as in Experimental Example 3, except that the incubation was performed for 6 hours after the addition of the FM medium and then the incubation was performed for 4 hours after the addition of the GM medium. In addition, the donor of the primary human hepatocyte used for the production of the liver organoid was a donor 2.

**[Table 15]**

| Condition | Glucagon | Cortisol | Forskolin | Recombinant human FGF10 | Recombinant human EGF | Recombinant human HGF | Insulin |
|---|---|---|---|---|---|---|---|
| 1 | - | - | - | - | - | - | - |
| 2 | - | 00 ng/mL | - | - | - | - | - |
| 3 | 200 pg/mL | 100 ng/mL | - | - | - | - | - |
| 4 | 200 ng/mL | 100 ng/mL | - | - | - | - | - |
| 5 | 200 pg/mL | 400 ng/mL | - | - | - | - | - |
| 6 | 200 ng/mL | 400 ng/mL | - | - | - | - | - |
| 7 | - | 400 ng/mL | 10 µM | - | - | - | - |
| 8 | 200 pg/mL | 1,000 ng/mL | - | - | - | - | - |
| 9 | 200 ng/mL | 1,000 ng/mL | - | - | - | - | - |
| 10 | 200 pg/mL | 400 ng/mL | - | 100 ng/mL | 50 ng/mL | 25 ng/mL | - |
| 11 | 200 pg/mL | 400 ng/mL | - | - | - | - | 1 µM |

Subsequently, the expression of various genes in each of the produced liver organoids was analyzed by quantitative real-time PCR (qPCR). Specifically, a total RNA was extracted from cells using Direct-zol RNA Kit micro (Zymo Research, catalog number: #R2063), and a reverse transcription reaction was carried out using a PrimeScript RT Master Mix (Takara Bio, catalog number: RR036A) to obtain a cDNA. Subsequently, quantitative real-time PCR was performed using LightCycler 480 System (Roche) and using TB Green Premix Ex Taq II (Takara Bio, catalog number: #RR820A). The cDNA of the primary human hepatocyte was diluted to create a calibration curve. The base sequences of the primers used are shown in Table 16.

**[Table 16]**

| Gene | Related function | Orientation | Base sequence (5'-3') | Sequence number |
|---|---|---|---|---|
| FBPl | Gluconeogenesis | Forward | CGCGCACCTCTATGGCATT | 5 |
| | | Reverse | TTCTTCTGACACGAGAACACAC | 6 |
| PCK1 | Gluconeogenesis | Forward | AAAACGGCCTGAACCTCTCG | 7 |
| | | Reverse | ACACAGCTCAGCGTTATTCTC | 8 |
| ASS1 | Urea production | Forward | TCCGTGGTTCTGGCCTACA | 9 |
| | | Reverse | GGCTTCCTCGAAGTCTTCCTT | 10 |
| CPS1 | Urea production | Forward | AATGAGGTGGGCTTAAAGCAAG | 11 |
| | | Reverse | AGTTCCACTCCACAGTTCAGA | 12 |
| SOCS2 | Growth hormone signaling | Forward | CAGATGTGCAAGGATAAGCGG | 13 |
| | | Reverse | GCGGTTTGGTCAGATAAAGGTG | 14 |

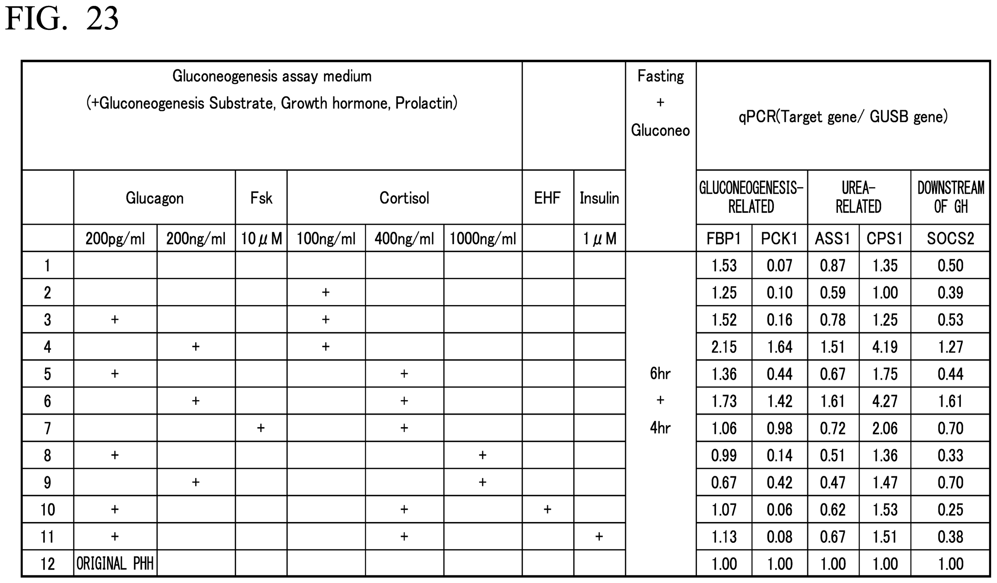

FIG. 23 is a table showing the results of qPCR. In FIG. 23, the numbers 1 to 11 correspond to the numbers 1 to 11 in Table 15. The number 12 in FIG. 23 is the result of the primary human hepatocyte used for the calibration curve of qPCR. As a result, it was revealed that the expression of the gluconeogenesis-related genes and the urea production-related genes was most increased in the GM medium including 200 ng/mL glucagon and 100 ng/mL cortisol.

In addition, it was revealed that the expression of the gluconeogenesis-related genes and the urea production-related genes was increased in the GM medium including 200 ng/mL glucagon and 400 ng/mL cortisol. In addition, it has also been revealed that insulin suppresses the expression of the gluconeogenesis-related genes.

### [Experimental Example 15]

(Examination 2 on Optimum Concentration of Composition of Gluconeogenesis Medium)

The gluconeogenesis of the liver organoid was evaluated in the same manner as in Experimental Example 3, except that the concentrations of various added factors were changed for the composition of the gluconeogenesis medium (GM medium). A GM medium in which the addition concentrations of the growth hormone and prolactin were changed to the conditions shown in Table 17 below from the GM medium having the composition shown in Table 4 was used for culture. In addition, the donor of the primary human hepatocyte used for the production of the liver organoid was a donor 9.

**[Table 17]**

| Condition | Growth hormone (ng/mL) | Prolactin (ng/mL) |
|---|---|---|
| 1 | 0 | 10 |
| 2 | 0.01 | 10 |
| 3 | 0.1 | 10 |
| 4 | 1 | 10 |
| 5 | 100 | 10 |
| 6 | 1,000 | 10 |
| 7 | 10 | 0 |
| 8 | 10 | 0.01 |
| 9 | 10 | 0.1 |
| 10 | 10 | 1 |
| 11 | 10 | 100 |
| 12 | 10 | 1,000 |

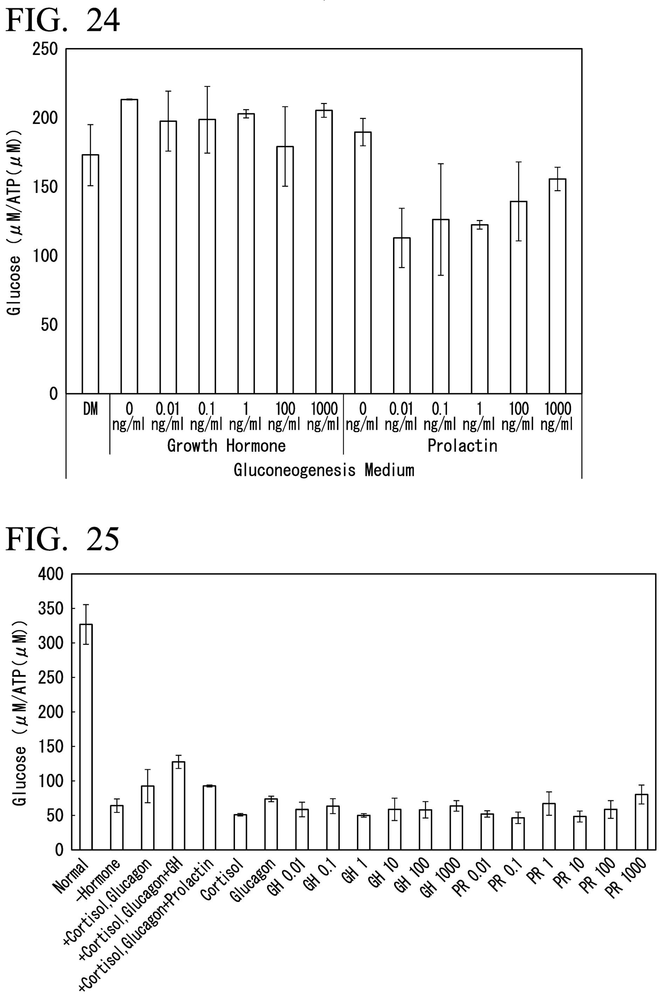

FIG. 24 is a graph showing the quantification results of glucose. In FIG. 24, "DM" indicates a differentiation medium. The numerical values shown in FIG. 24 are numerical values corrected by the amount of ATP measured by 3D-Cell Titer Glo assay.

### [Experimental Example 16]

### (Examination 3 on Optimum Concentration of Composition of Gluconeogenesis Medium)

The gluconeogenesis of the liver organoid was evaluated in the same manner as in Experimental Example 3, except that the concentrations of various added factors were changed for the composition of the gluconeogenesis medium (GM medium). A GM medium obtained by removing the growth hormone, prolactin, cortisol, and glucagon from the gluconeogenesis medium (GM medium) having the composition shown in Table 4, and further adding the components shown in Table 18 below was prepared. The gluconeogenesis of the liver organoid was evaluated in the same manner as in Experimental Example 3, except that the GM medium was used. In addition, the donor of the primary human hepatocyte used for the production of the liver organoid was a donor 9.

**[Table 18]**

| Condition | Growth hormone (ng/mL) | Prolactin (ng/mL) | Cortisol (ng/mL) | Glucagon (ng/mL) |
|---|---|---|---|---|
| 1 | - | - | - | - |
| 2 | - | - | 100 | 200 |
| 3 | 10 | - | 100 | 200 |
| 4 | - | 10 | 100 | 200 |
| 5 | - | - | 100 | - |
| 6 | - | - | - | 200 |
| 7 | 0.01 | - | - | - |
| 8 | 0.1 | - | - | - |
| 9 | 1 | - | - | - |
| 10 | 100 | - | - | - |
| 11 | 1,000 | - | | - |
| 12 | - | 0.01 | - | - |
| 13 | - | 0.1 | - | - |
| 14 | - | 1 | - | - |
| 15 | - | 100 | - | - |
| 16 | - | 1,000 | - | - |

FIG. 25 is a graph showing the quantification results of glucose. The numerical values shown in FIG. 25 are numerical values corrected by the amount of ATP measured by 3D-Cell Titer Glo assay. In FIG. 25, "GH" indicates a growth hormone, "PR" indicates prolactin, and "-Hormone" indicates that a growth hormone, prolactin, cortisol, and glucagon are not included.

As a result of FIGS. 24 and 25, it was revealed that the gluconeogenesis of the liver organoid is improved by using a gluconeogenesis medium including at least one of a growth hormone, prolactin, cortisol, and glucagon.

### [Experimental Example 17]

### (Examination on Transforming Growth Factor β Receptor Antagonist in Differentiation Induction of Liver Organoid)

A liver organoid was produced in the same manner as in Experimental Example 3, except that the transforming growth factor β receptor antagonist (A83-01) was removed from the differentiation medium (DM medium), and the expression of the gluconeogenesis-related genes, the urea production-related genes, and the CYP genes was examined by quantitative real-time PCR.

A total RNA was extracted from cells using Direct-zol RNA Kit micro (Zymo Research, catalog number: #R2063), and a reverse transcription reaction was carried out using a PrimeScript RT Master Mix (Takara Bio, catalog number: RR036A) to obtain a cDNA. Subsequently, quantitative real-time PCR was performed using LightCycler 480 System (Roche) and using TB Green Premix Ex Taq II (Takara Bio, catalog number: #RR820A). The cDNA of the primary human hepatocyte was diluted to create a calibration curve. The base sequences of the primers used are shown in Table 19 below.

**[Table 19]**

| Gene | Related function | Orientation | Base sequence (5'-3') | Sequence number |
|---|---|---|---|---|
| CYP2A6 | Drug metabolism | Forward | CTCATGAAGATCAGTGAGCGCTAT | 1 |
| | | Reverse | CTCCCCGTTGCTGAATACCA | 2 |
| CYP3A4 | Drug metabolism | Forward | TATGGAAAAGTGTGGGGCTT | 3 |
| | | Reverse | TCCGGTTTGTGAAGACAGAAT | 4 |
| FBP1 | Gluconeogenesis | Forward | CGCGCACCTCTATGGCATT | 5 |
| | | Reverse | TTCTTCTGACACGAGAACACAC | 6 |
| PCK1 | Gluconeogenesis | Forward | AAAACGGCCTGAACCTCTCG | 7 |
| | | Reverse | ACACAGCTCAGCGTTATTCTC | 8 |
| ASS1 | Urea production | Forward | TCCGTGGTTCTGGCCTACA | 9 |
| | | Reverse | GGCTTCCTCGAAGTCTTCCTT | 10 |
| CPS1 | Urea production | Forward | AATGAGGTGGGCTTAAAGCAAG | 11 |
| | | Reverse | AGTTCCACTCCACAGTTCAGA | 12 |

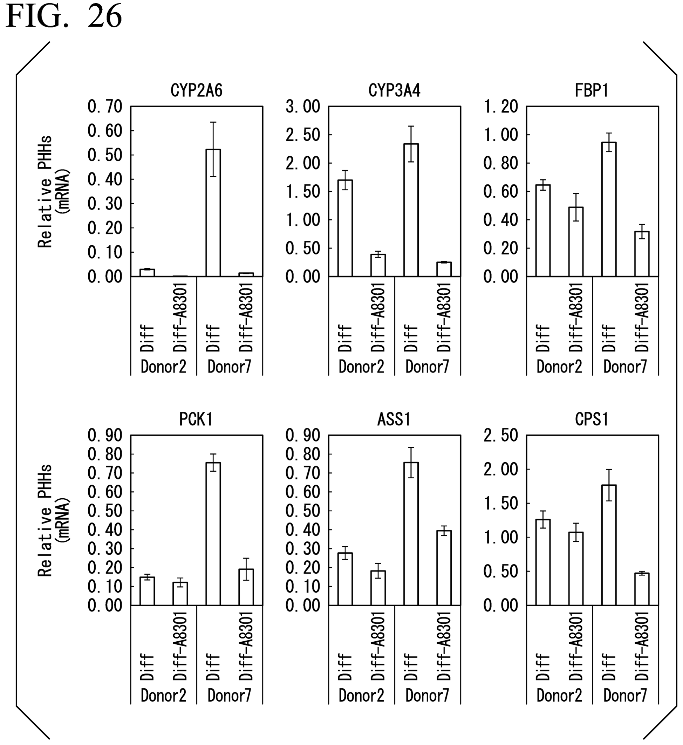

FIG. 26 is a graph showing the results of quantitative real-time PCR. In FIG. 26, "Diff" indicates the results of the liver organoids cultured in the DM medium, and "Diff-A8301" indicates the results of the liver organoids cultured in the DM medium from which A83-01 had been removed. In addition, the vertical axis of the graph indicates a relative value in which the expression level in the primary human hepatocytes (PHHs) is set to 1.

As a result, it was revealed that the expression of the gluconeogenesis-related genes, the urea production-related genes, and the CYP genes was decreased in the liver organoids cultured in the DM medium from which A83-01 had been removed.

### [Experimental Example 18]

### (Evaluation of Secreted Proteins)

In Experimental Examples 1 to 3, the amounts of albumin, complement C3, α1-antitrypsin, and a blood coagulation factor IX, which were secreted proteins included in the culture supernatant after culturing in the MM medium, were measured by Human Albumin ELISA Quantitation Kit (Bethyl Laboratories), Human alpha-1-antitrypsin AssayMax ELISA Kit (Assaypro), Human Complement C3 ELISA Kit (Abcam), and Human Factor IX ELISA Kit (Abcam), respectively. The measurement results are shown in FIG. 20.

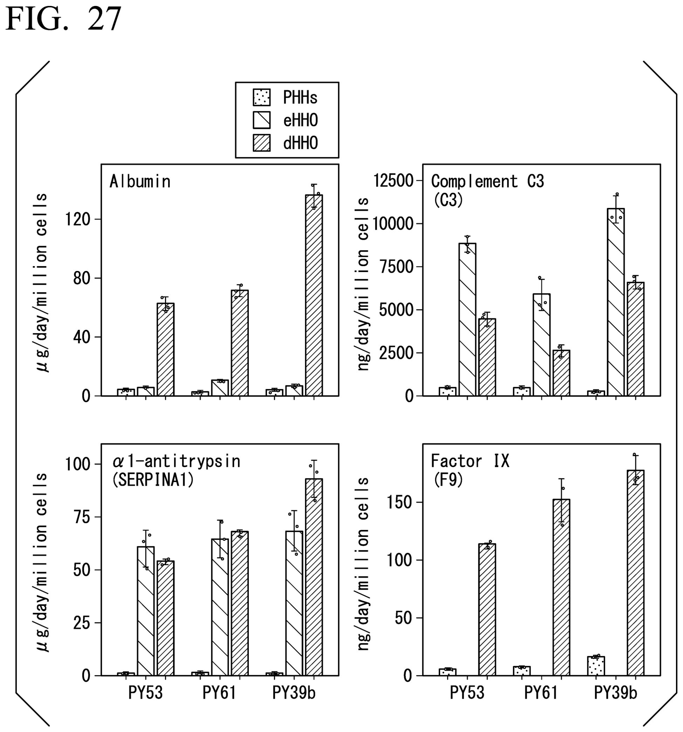

In FIG. 27, "PHHs" indicates measurement results of the primary human hepatocytes in Experimental Example 1, "eHHO" indicates the measurement results of the liver organoids in Experimental Example 2, and "dHHO" indicates the measurement results of the liver organoids in Experimental Example 3. In addition, with regard to the donor of the primary human hepatocyte used for the production of the liver organoid, "PY53" indicates the donor 2, "PY61" indicates the donor 3, and "PY39" indicates the donor 5. The data are expressed in a mean value ± a standard deviation.

It was revealed that the liver organoid obtained by culturing in the EM medium secretes a complement C3, which is a complement system protein that plays an important role in the immune response, and α1-antitrypsin that has an action of inhibiting a proteolytic enzyme. Furthermore, it has been shown that only the liver organoid obtained by culturing in a DM medium is capable of secreting a blood coagulation factor IX that plays a role in the formation of stabilized fibrin.

### [Experimental Example 19]

### (Evaluation of Amount of Lipid Produced)

The liver organoids in Experimental Examples 2 and 3 were cultured in an MM medium for 2 days, and the amount of lipid produced by the liver organoids (the amount of lipoprotein produced) was measured. In addition, in Experimental Example 3, the same measurement was performed on a liver organoid cultured by supplementing the DM medium with Wnt3a and R-Spondin1.

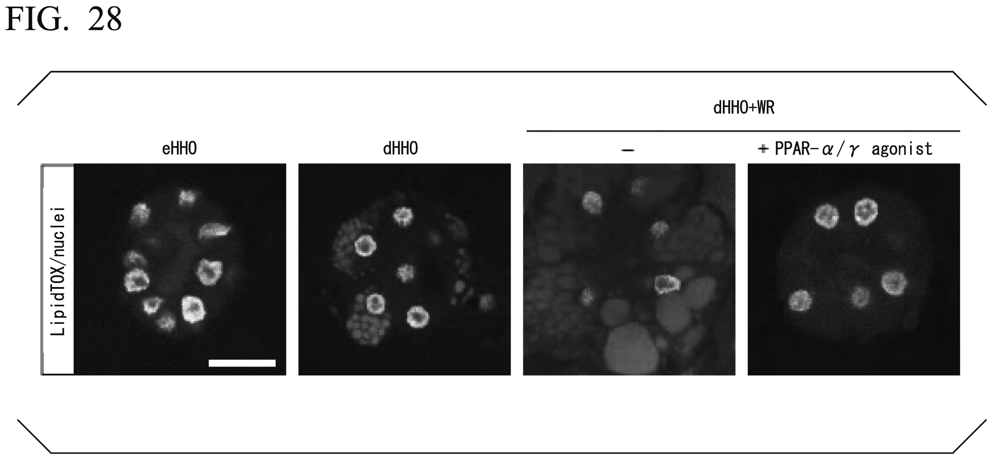

FIG. 28 is an image showing the results of lipid staining of the liver organoid. In FIG. 28, "eHHO" indicates the measurement results of the liver organoids in Experimental Example 2, and "dHHO" indicates the measurement results of the liver organoids in Experimental Example 3. In addition, "+WR" indicates the results of a liver organoid cultured by supplementing the DM medium with Wnt3a and R-Spondin1.

Lipid staining was performed using HCS LipidTOX^{™} Deep Red Neutral Lipid Stain, for cellular imaging (Thermo Fisher Scientific).

The amount of the lipid accumulated in the cell was measured from the lipid-stained image of the section. Image processing software (Image-J) was used for the measurement. The amount was measured as a volume per number of cells (nuclei) based on a three-dimensional reconstructed image acquired by a tissue three-dimensional structure construction method.

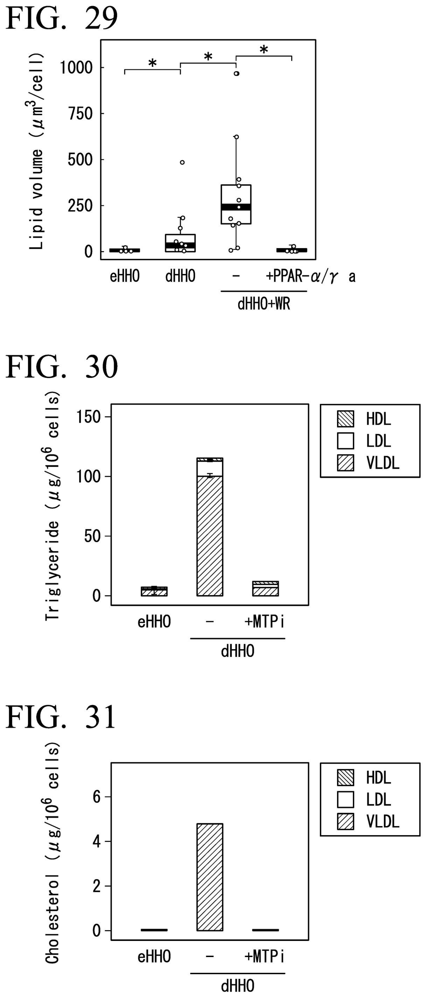

The measurement results of the amount of the lipid accumulated in the cell are shown in FIG. 29. In FIG. 29, "+PPAR-α/γ a" indicates the results of addition of a low-molecular-weight compound WY-16463 which is a PPAR-α/γ agonist. WY-16463 is known to be a ligand for a peroxisome proliferator-activated receptor (PPAR-α/γ) and to lead to a decrease in blood triglyceride concentration.

FIG. 30 is a graph showing the results of lipoprotein profiling performed in a culture supernatant by gel permeation HPLC of triglycerides. FIG. 31 is a graph showing the results of lipoprotein profiling performed in a culture supernatant by gel permeation HPLC of cholesterol.

In FIGS. 30 and 31, "HDL" indicates high-density lipoprotein, "LDL" indicates low-density lipoprotein, and "VLDL" indicates very-low-density lipoprotein. In addition, "+MTPi" indicates that a low-molecular-weight compound called Lomitapide was added. Lomitapide is a microsomal triglyceride transfer protein (MTP) inhibitor, and is known to selectively inhibit MTP involved in the synthesis and secretion of lipoproteins including apolipoprotein B in the liver and the small intestine, and to suppress the production of VLDL and the synthesis of LDL, reducing LDL cholesterol.

### [Experimental Example 20]

### (Evaluation of Glycogen)

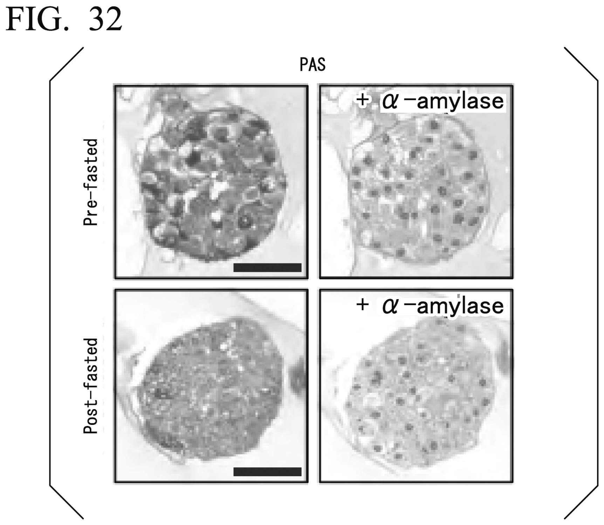

FIG. 32 is an image showing the results of staining the liver organoid with periodic acid-Schiff (PAS, Sigma-Aldrich) before and after the fasting in Experimental Example 3. Glycogen can be stained by PAS staining. In FIG. 32, "+ α-amylase" indicates the results of addition of amylase. It was confirmed that glycogen was stained by PAS staining since the addition of amylase reduced the staining intensity.

As a result, it was shown that the glycogen of the liver organoid was reduced by fasting.

### [Experimental Example 21]

### (Toxicity Evaluation of Acetaminophen)

The toxicity of acetaminophen was evaluated using a liver organoid produced in the same manner as in Experimental Example 3. The donor of the primary human hepatocyte used for the production of the liver organoid was a donor 10.

Acetaminophen was added to a differentiation medium (DM medium) to final concentrations of 0, 0.3 mM, 0.5 mM, 1 mM, 5 mM, and 10 mM, and the liver organoid was cultured for 24 hours. Thereafter, the amount of ATP was measured by 3D-Cell Titer Glo assay using the cultured cells. In addition, the culture supernatant was used to quantify lactate dehydrogenase (LDH) by LDH glo assay. The measurement by 3D-Cell Titer Glo assay was performed according to a protocol of the manufacturer using CellTiter-Glo (R) 3D Cell Viability Assay (Promega Corporation, G9682). The LDH glo assay was carried out according to a protocol of the manufacturer using LDH-Glo^{™} Cytotoxicity Assay (Promega Corporation, J2381).

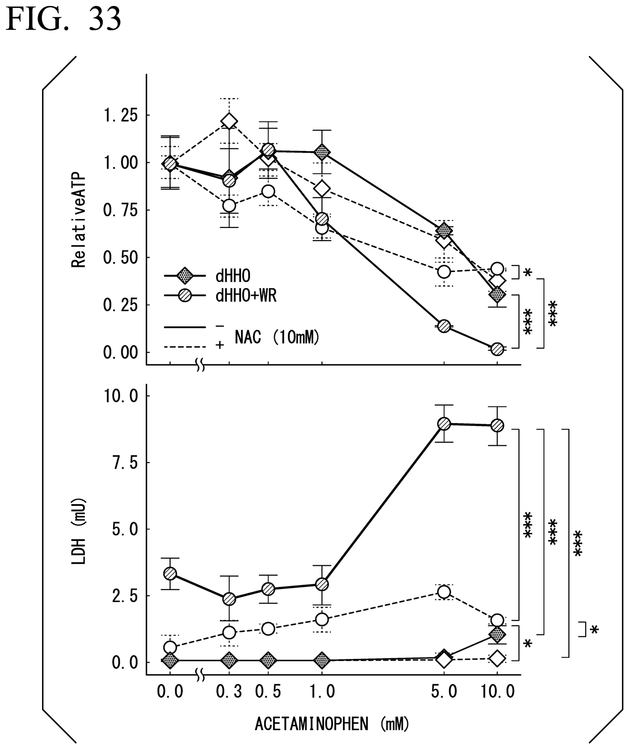

The upper part of FIG. 33 is a graph showing the measurement results of the amount of ATP. The vertical axis of the graph indicates the amount of ATP (relative value), and the horizontal axis of the graph indicates the concentration of acetaminophen added. The lower part of FIG. 33 is a graph showing the measurement results of the amount of LDH. The vertical axis of the graph indicates the amount of LDH, and the horizontal axis of the graph indicates the concentration of acetaminophen added. In FIG. 33, "dHHO" indicates the measurement results of a liver organoid produced in the same manner as in Experimental Example 3, and "+WR" indicates the measurement results of a liver organoid produced in the same manner as in Experimental Example 3, except that the DM medium was supplemented with 20 v/v% Wnt3a conditioned medium and 5 v/v% R-Spondin1 conditioned medium. Furthermore, "-NAC" indicates the results of a liver organoid cultured without adding N-acetyl-L-cysteine (NAC) to "dHHO" or "+WR", and "+NAC (10 mM)" indicates the results of a liver organoid cultured by adding NAC to "dHHO" or "+WR" at 10 mM. In addition, "*" indicates that there is a significant difference at p < 0.05, and "***" indicates that there is a significant difference at p < 0.001.

As a result, it was shown that the exposure of the produced liver organoid to acetaminophen resulted in a decrease in ATP and an increase in LDH in a concentration-dependent manner of acetaminophen.

### [Experimental Example 22]

### (Functional Evaluation of Bile Acid Transporter)

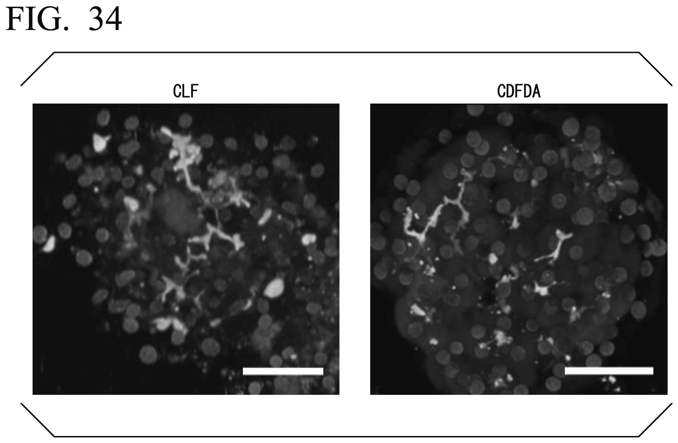

The left side of FIG. 34 is a fluorescence image showing the results of observation 60 minutes after adding 1 µg/mL of CLF (Corning, 451041), which is a fluorescent substrate of BSEP as a bile acid transporter, to the medium of a liver organoid produced in the same manner as in Experimental Example 3. The scale bar is 50 µm. The donor of the primary human hepatocyte used for the production of the liver organoid was a donor 11.

The right side of FIG. 34 is a fluorescence image showing the results of observation 60 minutes after adding 10 µM of CDFDA (Sigma-Aldrich, 21884), which is a fluorescent substrate of MRP2 as a bile acid transporter, to the medium of a liver organoid produced in the same manner as in Experimental Example 3. The scale bar is 50 µm. The donor of the primary human hepatocyte used for the production of the liver organoid was a donor 11.

As a result, it was shown that a liver organoid produced in the same manner as in Experimental Example 3 had the activities of BSEP and MRP2.

### [Industrial Applicability]

According to the present invention, it is possible to provide a technique for producing a metabolically activated liver organoid, in which gluconeogenesis and urea production are activated.

## Claims

1. A method for producing a metabolically activated liver organoid, the method comprising:
a step (a) of culturing primary hepatocytes or hepatocyte-like cells in a differentiation medium to obtain a metabolically activated liver organoid,
wherein the differentiation medium is a medium including one or two selected from the group consisting of a growth hormone receptor agonist and a prolactin receptor agonist, and a glucocorticoid receptor agonist.

2. The method for producing a metabolically activated liver organoid according to Claim 1,
wherein the differentiation medium includes the growth hormone receptor agonist and the prolactin receptor agonist.

3. The method for producing a metabolically activated liver organoid according to Claim 1 or 2,
wherein the differentiation medium further includes one or two growth factors selected from the group consisting of an epidermal growth factor (EGF), a hepatocyte growth factor (HGF), and a fibroblast growth factor (FGF).

4. The method for producing a metabolically activated liver organoid according to Claim 1 or 2,
wherein the differentiation medium further includes a transforming growth factor β receptor antagonist.

5. The method for producing a metabolically activated liver organoid according to Claim 1 or 2,
wherein the differentiation medium further includes a γ-secretase inhibitor.

6. The method for producing a metabolically activated liver organoid according to Claim 1 or 2,
wherein the differentiation medium further includes one or two or more selected from the group consisting of estradiol, dehydroepiandrosterone, and glucagon.

7. The method for producing a metabolically activated liver organoid according to Claim 1 or 2, the method further comprising, in the following order after the step (a):
a step (b) of fasting the metabolically activated liver organoid; and
a step (c) of culturing the metabolically activated liver organoid in a gluconeogenesis medium,
wherein the gluconeogenesis medium is a medium including a gluconeogenesis substrate.

8. The method for producing a metabolically activated liver organoid according to Claim 7,
wherein the gluconeogenesis medium further includes a glucagon receptor agonist.

9. The method for producing a metabolically activated liver organoid according to Claim 7,
wherein the gluconeogenesis medium further includes one or two selected from the group consisting of a growth hormone receptor agonist and a prolactin receptor agonist, and a glucocorticoid receptor agonist.

10. A composition comprising:
one or two selected from the group consisting of a growth hormone receptor agonist and a prolactin receptor agonist; and
a glucocorticoid receptor agonist.

11. A method for evaluating a test substance, the method comprising:
a step of bringing a test substance into contact with a metabolically activated liver organoid produced by the production method according to Claim 1 or 2; and
a step of evaluating an effect of the test substance on the metabolically activated liver organoid.
